(19) Europäisches Patentamt
European Patent Office
Office européen des brevets

(11) **EP 3 932 302 B1**

(12) **EUROPEAN PATENT SPECIFICATION**

(45) Date of publication and mention
of the grant of the patent:
**16.10.2024 Bulletin 2024/42**

(51) International Patent Classification (IPC):
*A61B 5/026* (2006.01)    *A61M 60/109* (2021.01)
*A61M 1/36* (2006.01)    *A61M 60/232* (2021.01)
*A61M 60/523* (2021.01)    *A61M 60/546* (2021.01)
*A61M 60/585* (2021.01)

(21) Application number: **20831956.6**

(52) Cooperative Patent Classification (CPC):
**A61B 5/4836; A61B 5/026; A61M 1/3666;**
**A61M 60/109; A61M 60/232; A61M 60/38;**
**A61M 60/523; A61M 60/546; A61M 60/585;**
A61M 2205/3334; A61M 2205/502

(22) Date of filing: **31.03.2020**

(86) International application number:
**PCT/JP2020/014809**

(87) International publication number:
**WO 2020/261688 (30.12.2020 Gazette 2020/53)**

(54) **CARDIAC FUNCTION MEASUREMENT SYSTEM, EXTRACORPOREAL CIRCULATION DEVICE, AND CARDIAC FUNCTION MEASUREMENT PROGRAM**

HERZFUNKTIONSMESSSYSTEM, EXTRAKORPORALE ZIRKULATIONSVORRICHTUNG UND HERZFUNKTIONSMESSPROGRAMM

SYSTÈME DE MESURE DE FONCTION CARDIAQUE, DISPOSITIF DE CIRCULATION EXTRA-CORPORELLE ET PROGRAMME DE MESURE DE FONCTION CARDIAQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2019 JP 2019119835**

(43) Date of publication of application:
**05.01.2022 Bulletin 2022/01**

(73) Proprietor: **TERUMO Kabushiki Kaisha**
**Tokyo 151-0072 (JP)**

(72) Inventors:
• **UTSUGIDA Tomoki**
**Ashigarakami-gun Kanagawa 259-0151 (JP)**
• **CHINO Naotaka**
**Ashigarakami-gun Kanagawa 259-0151 (JP)**
• **SUGIYAMA Yuichiro**
**Tokyo 163-1450 (JP)**
• **YAMADA Makoto**
**Tokyo 163-1450 (JP)**

(74) Representative: **Casalonga**
**Casalonga & Partners**
**Bayerstraße 71/73**
**80335 München (DE)**

(56) References cited:
WO-A1-2017/115487    JP-A- 2017 217 296
JP-A- 2017 217 296    US-A1- 2018 353 667

**Description**

Technical Field

This disclosure relates to a cardiac function measurement system, an extracorporeal circulator, and a cardiac function measurement program. In particular, the present invention relates to a cardiac function measurement system according to the preamble of claim 1, such as it is e.g.known from US 2018/353667 A1.

Background Art

As one of professional resuscitation treatments for cardiogenic cardiac arrest patients, there is an invasive percutaneous cardiopulmonary support (PCPS) using an extracorporeal circulator as a percutaneous cardiopulmonary auxilairy device. Such a resuscitation treatment may be referred to as extracorporeal cardiopulmonary resuscitation (ECPR) or extracorporeal membrane oxygenation (ECHO).

For the invasive percutaneous cardiopulmonary support using the extracorporeal circulator, one of important factors is to determine whether a cardiac function (state of the heart) of a patient is recovered. That is, when a withdrawal timing of the extracorporeal circulator is too early with respect to the recovery of the cardiac function of the patient, the heart of the patient cannot stand the withdrawal timing, and it may be necessary to reattach the extracorporeal circulator or the patient may die. On the other hand, when the withdrawal timing of the extracorporeal circulator is too late with respect to the recovery of the cardiac function of the patient, complications such as bleeding may occur due to anticoagulant therapy, consumption of a blood plasma component, or the like. That is, there is an appropriate timing for the withdrawal of the extracorporeal circulator. Further, it is desirable that the withdrawal timing of the extracorporeal circulator can be early ascertained.

During the invasive percutaneous cardiopulmonary support using the extracorporeal circulator, a pump of the extracorporeal circulator removes blood from the patient, causes the blood to pass through an oxygenator, and returns the blood that has passed through the oxygenator to the patient. At this time, a direction of a flow of the blood returned into the body of the patient by the pump is opposite to a direction of a flow of the blood flowing through the body of the patient. That is, the pump returns the blood that has passed through the oxygenator to the patient in a state in which the blood flows retrogradely. Therefore, even in a case in which a flow rate sensor attached to a catheter is delivered to the vicinity of the heart of the patient, it is difficult to only measure a cardiac output. Therefore, in this case, it is difficult to determine the recovery of the cardiac function of the patient with high accuracy.

During the invasive percutaneous cardiopulmonary support using the extracorporeal circulator, there are relatively many facilities in which the flow rate sensor attached to the catheter is not delivered to the vicinity of the heart of the patient. Therefore, also in this case, it is difficult to determine the recovery of the cardiac function of the patient with high accuracy. In order to determine the recovery of the cardiac function, a facility is also present that temporarily stops operations of the extracorporeal circulator and measures the cardiac output. However, in order to reduce a burden on the patient, it is desirable that the recovery of the cardiac function can be determined not intermittently but continuously.

PTL 1 discloses a cardiac function evaluation apparatus that calculates a time differential of a blood flow rate measured by a blood flowmeter and evaluates a cardiac function based on a result of the calculation. The cardiac function evaluation apparatus disclosed in PTL 1 converts the time differential of the blood flow rate into a time differential of a blood pressure. A relationship between the time differential of the blood flow rate and the time differential of the blood pressure is shown in Fig. 2 in PTL 1. According to Fig. 2 described in PTL 1, an error of the time differential of the blood pressure is relatively large even though a cardiac function evaluation block including the blood flowmeter is disposed in the vicinity of the heart of the patient. Therefore, as described above, even when the blood flowmeter is disposed in the vicinity of the heart of the patient, it is difficult to determine the recovery of the cardiac function of the patient with high accuracy.

Citation List

Patent Literature

PTL 1: JP-A-2002-224066

Summary of Invention

Technical Problem

This disclosure is made to solve the above problem, and an object of this disclosure is to provide a cardiac function measurement system, an extracorporeal circulator, and a cardiac function measurement program that are capable of determining recovery of a cardiac function of a patient with high accuracy.

Solution to Problem

According to the invention, the above problem is solved by a cardiac function independent claim 1. The dependent claims relate to advantageous embodiments. Claim 7 furthermore provides a corresponding cardiac function measurement program executed by a computer.

According to the above, the cardiac function measurement system according to this disclosure determines the withdrawal reference flow rate indicating the theoretical flow rate of the blood transferred from the pump based on the discharge pressure of the pump determined based on the rotation speed of the pump. The cardiac function measurement system determines the measured blood transfer flow rate indicating the realistic flow rate of the blood based on the measurement result of the flow rate measurement unit configured to measure the flow rate of the blood flowing through the circulation circuit of the extracorporeal circulator. Then, the cardiac function measurement system determines the withdrawal timing of the extracorporeal circulator based on the comparison between the withdrawal reference flow rate and the measured blood transfer flow rate. In this manner, the cardiac function measurement system according to this disclosure compares the withdrawal reference flow rate with the measured blood transfer flow rate having a relatively small temporal change, and determines the withdrawal timing of the extracorporeal circulator. Accordingly, the cardiac function measurement system according to this disclosure can determine the recovery of the cardiac function of the patient with high accuracy.

According to the cardiac function measurement system in this disclosure, by calculating the parameter indicating the comparison between the withdrawal reference flow rate and the measured blood transfer flow rate, an output ability of the patient can be quantitatively measured, and the withdrawal timing of the extracorporeal circulator can be determined based on the quantitative comparison. Accordingly, the cardiac function measurement system according to this disclosure can determine the recovery of the cardiac function of the patient with high accuracy.

By using a relatively simple numerical formula, a table, or the like, the cardiac function measurement system according to this disclosure can calculate the difference parameter representing the difference between the withdrawal reference flow rate and the measured blood transfer flow rate as the parameter indicating the comparison between the withdrawal reference flow rate and the measured blood transfer flow rate, and can quantitatively measure the output ability of the patient. Accordingly, the cardiac function measurement system according to this disclosure can easily determine the recovery of the cardiac function of the patient with high accuracy.

In the cardiac function measurement system according to this disclosure, it is preferable that a graph representing a relationship between the parameter and an elapsing time and the parameter are displayed on a display unit.

According to the cardiac function measurement system in this disclosure, by checking the display unit, an operator or the like can easily compare the withdrawal reference flow rate and the measured blood transfer flow rate, and can visually grasp the withdrawal timing of the extracorporeal circulator.

In the cardiac function measurement system according to this disclosure, it is preferable that, a graph representing a relationship between the rotation speed, and the withdrawal reference flow rate and the measured blood transfer flow rate is displayed on the display unit.

According to the cardiac function measurement system in this disclosure, by checking the display unit, an operator or the like can easily compare the withdrawal reference flow rate and the measured blood transfer flow rate, and can visually grasp the withdrawal timing of the extracorporeal circulator.

According to the cardiac function measurement system in this disclosure, the operator or the like can visually and easily grasp whether the withdrawal timing of the extracorporeal circulator is appropriate by checking the notification displayed on the display unit. Accordingly, the cardiac function measurement system according to this disclosure can prevent the withdrawal timing of the extracorporeal circulator from being too earlier than the recovery of the cardiac function of the patient, and can efficiently assist the determination of the withdrawal timing of the extracorporeal circulator.

In the cardiac function measurement system according to this disclosure, it is preferable that, when a predetermined time elapses after the notification that the withdrawal timing is appropriate is displayed on the display unit, a notification that the withdrawal timing is late is displayed on the display unit.

According to the cardiac function measurement system in this disclosure, the operator or the like can visually and easily grasp whether the withdrawal timing of the extracorporeal circulator is late by checking the notification displayed on the display unit. Accordingly, the cardiac function measurement system according to this disclosure can prevent the withdrawal timing of the extracorporeal circulator from being too later than the recovery of the cardiac function of the patient, and can efficiently assist the determination of the withdrawal timing of the extracorporeal circulator.

In the cardiac function measurement system according to this disclosure, it is preferable that the measured blood transfer flow rate is an average flow rate indicating an average value of a plurality of the realistic flow rates measured by the flow rate measurement unit in a predetermined time.

According to this, the cardiac function measurement system according to this disclosure can determine the withdrawal timing of the extracorporeal circulator by comparing the withdrawal reference flow rate and the measured blood transfer flow rate with each other and quantitatively measuring the output ability of the patient while preventing an influence of pulsations of the heart having a relatively large temporal change. Accordingly, the cardiac function measurement system according to this disclosure can determine the recovery of the cardiac function of the patient with higher accuracy.

According to the extracorporeal circulator in this disclosure, the cardiac function measurement system determines the withdrawal reference flow rate indicating the theoretical flow rate of the blood transferred from the pump based on the discharge pressure of the pump determined based on the rotation speed of the pump. The cardiac function measurement system determines the measured blood transfer flow rate indicating the realistic flow rate of the blood based on the measurement result of the flow rate measurement unit configured to measure the flow rate of the blood flowing through the circulation circuit of the extracorporeal circulator. Then, the cardiac function measurement system determines the withdrawal timing of the extracorporeal circulator based on the comparison between the withdrawal reference flow rate and the measured blood transfer flow rate. In this manner, the cardiac function measurement system compares the withdrawal reference flow rate with the measured blood transfer flow rate having a relatively small temporal change, and determines the withdrawal timing of the extracorporeal circulator. Accordingly, the extracorporeal circulator according to this disclosure can determine the recovery of the cardiac function of the patient with high accuracy.

By executing the cardiac function measurement program according to this disclosure, the withdrawal reference flow rate indicating the theoretical flow rate of the blood transferred from the pump is determined based on the discharge pressure of the pump determined based on the rotation speed of the pump. By executing the cardiac function measurement program, the measured blood transfer flow rate indicating the realistic flow rate of the blood is determined based on the measurement result of the flow rate measurement unit configured to measure the flow rate of the blood flowing through the circulation circuit of the extracorporeal circulator. Then, by executing the cardiac function measurement program, the withdrawal timing of the extracorporeal circulator is determined based on the comparison between the withdrawal reference flow rate and the measured blood transfer flow rate. In this manner, by executing the cardiac function measurement program, the withdrawal reference flow rate is compared with the measured blood transfer flow rate having a relatively small temporal change, and the withdrawal timing of the extracorporeal circulator is determined. Accordingly, by executing the cardiac function measurement program in this disclosure, the recovery of the cardiac function of the patient can be determined with high accuracy.

Advantageous Effect of Invention

According to this disclosure, it is possible to provide a cardiac function measurement system, an extracorporeal circulator, and a cardiac function measurement program that are capable of determining recovery of a cardiac function of a patient with high accuracy.

Brief Description of Drawings

[Fig. 1] Fig. 1 is a schematic view showing an extracorporeal circulator according to an embodiment of this disclosure.
[Fig. 2] Fig. 2 is a schematic diagram illustrating a flow of blood returned from the extracorporeal circulator to a patient and a flow of blood transferred from the heart of the patient.
[Fig. 3] Fig. 3 is a block diagram illustrating a control unit and a flow rate measurement unit according to the present embodiment.
[Fig. 4] Fig. 4 is a block diagram illustrating a configuration of main units of a cardiac function measurement system according to the present embodiment.
[Fig. 5] Fig. 5 is a block diagram illustrating a configuration of main units of the cardiac function measurement system according to the present embodiment.
[Fig. 6] Fig. 6 is a graph showing an example of a pump characteristic stored in a pump characteristic storage unit according to the present embodiment.
[Fig. 7] Fig. 7 is a flowchart showing a specific example of operations of the cardiac function measurement system according to the present embodiment.
[Fig. 8] Fig. 8 is a flowchart showing a specific example of the operations of the cardiac function measurement system according to the present embodiment.
[Fig. 9] Fig. 9 is a schematic view showing a first specific example of a screen displayed on a display unit according to the present embodiment.
[Fig. 10] Fig. 10 is a schematic view showing a second specific example of the screen displayed on the display unit according to the present embodiment.

[Fig. 11] Fig. 11 is a block diagram illustrating a first circulation system set in a present experiment.

[Fig. 12] Fig. 12 is a block diagram illustrating a second circulation system set in the present experiment.

[Fig. 13] Fig. 13 is a graph showing an example of a relationship between a rotation speed of a centrifugal pump and a measured blood transfer flow rate obtained in the present experiment.

[Fig. 14] Fig. 14 is a graph showing an example of a parameter CP calculated by a parameter calculation unit in the present experiment.

Description of Embodiments

Hereinafter, a preferred embodiment of this disclosure will be described in detail with reference to the drawings.

The embodiment to be described below is a preferred specific example of this disclosure, and thus various technically preferable limitations are applied to the embodiment. However, unless there is any particular mention which limits this disclosure in the description below, the range of this disclosure is not limited to these aspects. In the drawings, the same components are denoted by the same reference numerals, and a detailed description thereof will be omitted as appropriate.

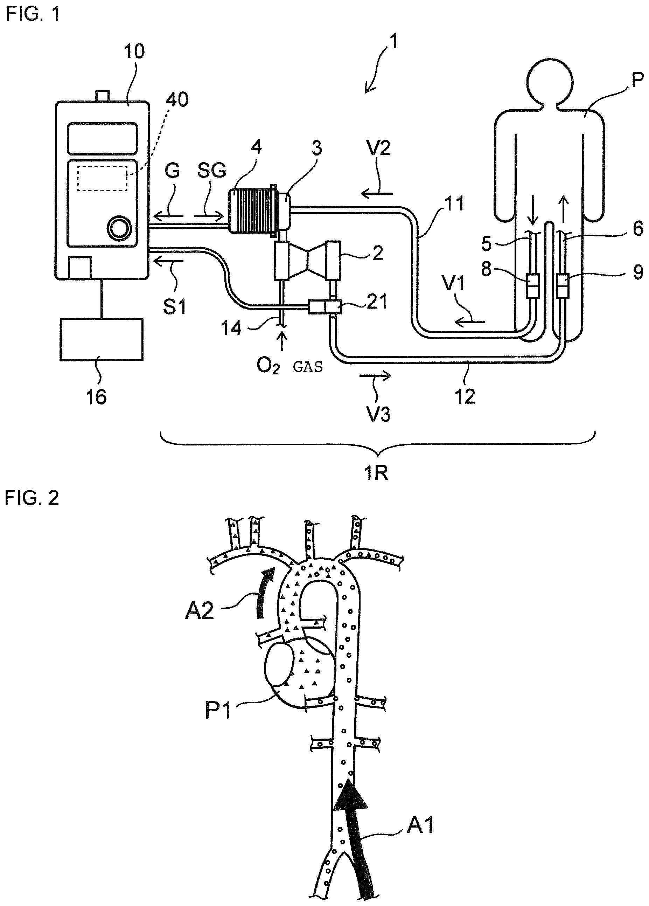

Fig. 1 is a schematic view showing an extracorporeal circulator according to the embodiment of this disclosure.

An "extracorporeal circulation" performed by an extracorporeal circulator 1 shown in Fig. 1 includes an "extracorporeal circulation operation" and an "auxiliary circulation operation". The extracorporeal circulator 1 can perform both the "extracorporeal circulation operation" and the "auxiliary circulation operation".

The "extracorporeal circulation operation" means that when blood circulation in the heart is temporarily stopped due to, for example, cardiac surgery, the extracorporeal circulator 1 performs a blood circulation operation and a gas exchange operation (oxygen addition and/or carbon dioxide removal) on the blood.

The "auxiliary circulation operation" means that the extracorporeal circulator 1 also performs the blood circulation operation and the gas exchange operation on the blood in a case in which the heart of a patient P cannot perform a sufficient function or in a state in which the lungs cannot perform gas exchange sufficiently. The extracorporeal circulator 1 is to be applied to the heart of the patient P. In the description of the present embodiment, the "auxiliary circulation operation" is mainly taken as an example.

For example, the extracorporeal circulator 1 is applied to a case in which the heart of the patient P does not operate normally, a case in which the heart of the patient P operates normally but the lungs do not operate normally, and the like. Accordingly, the extracorporeal circulator 1 shown in Fig. 1 is used, for example, in a case of performing the cardiac surgery on the patient P, in subsequent treatment in ICU, and the like. The extracorporeal circulator 1 shown in Fig. 1 performs oxygenator external blood circulation. In the oxygenator external blood circulation, a pump of the extracorporeal circulator 1 is operated to remove blood from a vein of a patient, the blood is subjected to the gas exchange by an oxygenator to perform oxygenation of the blood, and then the oxygenated blood is returned to an artery or the vein of the patient again. As described above, the extracorporeal circulator 1 is a device that functions as substitutes of the heart and the lung.

As shown in Fig. 1, the extracorporeal circulator 1 has a circulation circuit 1R that circulates blood. The circulation circuit 1R includes an oxygenator 2, a centrifugal pump 3, a drive motor 4 that drives the centrifugal pump 3, a blood removal side catheter (vein side catheter) 5, a blood transfer side catheter (artery side catheter) 6, and a cardiac function measurement system 10. The centrifugal pump 3 according to the present embodiment is an example of the "pump" according to this disclosure. The cardiac function measurement system 10 includes a control unit 40 and is provided as a controller of the extracorporeal circulator 1. The centrifugal pump 3 is also referred to as a blood pump or the like, and may be a pump other than the centrifugal pump.

The blood removal side catheter 5 is also referred to as a vein side cannula (blood removal side cannula) or the like, and is inserted from a femoral vein. A distal end of the blood removal side catheter 5 is indwelled in a right atrium. The blood removal side catheter 5 is connected to a blood removal tube (also referred to as a blood removal line) 11 via a connector 8, is connected to the centrifugal pump 3 via the blood removal tube 11, and guides the blood removed from the patient P to the centrifugal pump 3 via the blood removal tube 11. The blood removal tube 11 is a conduit that connects the blood removal side catheter 5 and the centrifugal pump 3, and is a conduit that guides the blood removed from the patient P to the centrifugal pump 3 via the blood removal side catheter 5.

The blood transfer side catheter 6 is also referred to as an artery side cannula (blood transfer side cannula) or the like, and is inserted from a femoral artery. The blood transfer side catheter 6 is connected to a blood transfer tube (also referred to as a blood transfer line) 12 via a connector 9, is connected to the oxygenator 2 via the blood transfer tube 12, and guides the blood that has passed through the oxygenator 2 to the patient P via the blood transfer tube 12. The blood transfer tube 12 is a conduit that connects the oxygenator 2 and the blood transfer side catheter 6, and is a conduit that guides the blood to the patient P. The blood has passed through the oxygenator 2.

The drive motor 4 controls driving of the centrifugal pump 3 based on a command SG of the cardiac function measurement system 10. The centrifugal pump 3 is provided downstream of the blood removal side catheter 5, and is driven by receiving a drive force transmitted from the drive motor 4. The centrifugal pump 3 removes the blood from the patient P via the blood removal side catheter 5 and the blood removal tube 11, transfers the blood to the oxygenator 2, and then returns the blood to the patient P via the blood transfer tube 12 and the blood transfer side catheter 6. The centrifugal pump 3 transmits a signal G related to a rotation speed of the centrifugal pump 3 to the cardiac function measurement system 10.

The oxygenator 2 is provided downstream of the centrifugal pump 3. Specifically, the oxygenator 2 is disposed between the centrifugal pump 3 and the blood transfer tube 12. The oxygenator 2 performs the gas exchange operation (oxygen addition and/or carbon dioxide removal) on the blood. The oxygenator 2 is, for example, a membrane-type oxygenator, and is particularly preferably a hollow fiber membrane-type oxygenator. Oxygen gas is supplied to the oxygenator 2 through an oxygen supply tube 14.

As the blood removal tube 11 and the blood transfer tube 12, for example, conduits made of a highly transparent, elastically deformable, and flexible synthetic resin such as vinyl chloride resin or silicone rubber are used. The blood, which is a liquid, flows in a V1 direction and a V2 direction in the blood removal tube 11, and flows in a V3 direction in the blood transfer tube 12.

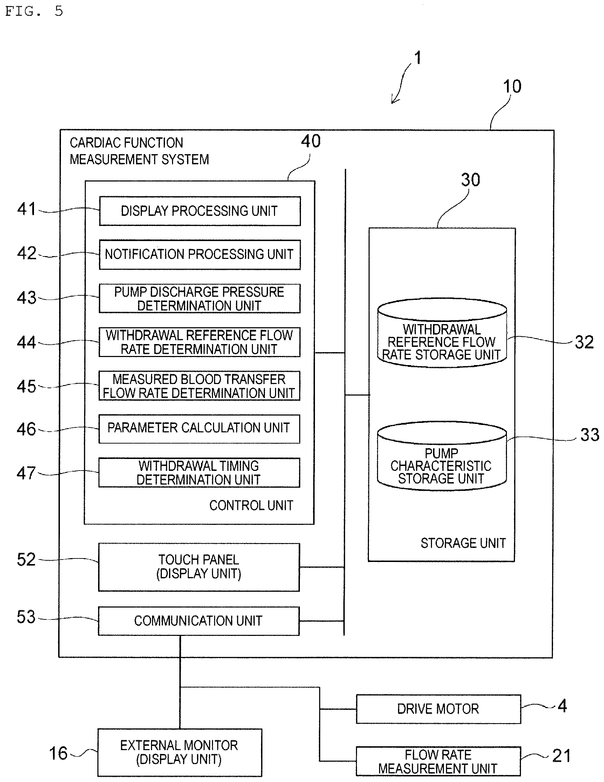

The cardiac function measurement system 10 acquires various types of information, executes calculation, generates a control signal for controlling operations of devices such as the drive motor 4 and an external monitor 16, and transmits the control signal to each device. In other words, the cardiac function measurement system 10 manages the extracorporeal circulator 1. Details of the cardiac function measurement system 10 will be described below. The cardiac function measurement system 10 may include a touch panel 52 (see Fig. 5) as an input unit capable of inputting various types of information and as a display unit that displays various types of information. That is, the "display unit" according to this disclosure may be the external monitor 16 provided separately from the cardiac function measurement system 10, or may be the touch panel 52 provided in the cardiac function measurement system 10. The touch panel 52 can detect contact, or the like, of a finger of an operator or the like.

The extracorporeal circulator 1 according to the present embodiment further includes a flow rate measurement unit 21 and the external monitor (display unit) 16. The external monitor 16 according to the present embodiment is an example of the "display unit" according to this disclosure. In the following description, a case in which the "display unit" according to this disclosure is the external monitor 16 will be described as an example.

The flow rate measurement unit 21 is provided in the circulation circuit 1R. In the extracorporeal circulator 1 according to the present embodiment, the flow rate measurement unit 21 is provided in the circulation circuit 1R between the oxygenator 2 and the blood transfer side catheter 6. Specifically, the flow rate measurement unit 21 is provided on the blood transfer tube 12. By providing the flow rate measurement unit 21 on the blood transfer tube 12, the flow rate measurement unit 21 can measure a flow rate of blood immediately before being returned to the patient P, and can measure a flow rate of blood flowing through the circulation circuit 1R at a position relatively close to the patient P. However, an arrangement position of the flow rate measurement unit 21 is not limited to the blood transfer tube 12, and may be any position in the circulation circuit 1R. In the following description, a case in which the flow rate measurement unit 21 is provided on the blood transfer tube 12 will be described as an example.

The flow rate measurement unit 21 is, for example, a flow rate sensor, and detects a flow rate of blood flowing inside the circulation circuit 1R. The flow rate measurement unit 21 shown in Fig. 1 measures a flow rate of the blood transferred from the centrifugal pump 3. The flow rate measurement unit 21 transmits a signal S1 related to the flow rate of the blood flowing inside the circulation circuit 1R to the cardiac function measurement system 10. In the present embodiment, the flow rate measurement unit 21 transmits, to the cardiac function measurement system 10, the signal S1 related to the flow rate of the blood transferred from the centrifugal pump 3. Examples of the flow rate measurement unit 21 includes an ultrasound flowmeter. As the ultrasound flowmeter, for example, an ultrasound propagation time difference type flowmeter is used. However, the flow rate measurement unit 21 is not limited to the ultrasound flowmeter.

Fig. 2 is a schematic diagram illustrating a flow of blood returned from the extracorporeal circulator to a patient and a flow of blood transferred from the heart of the patient.

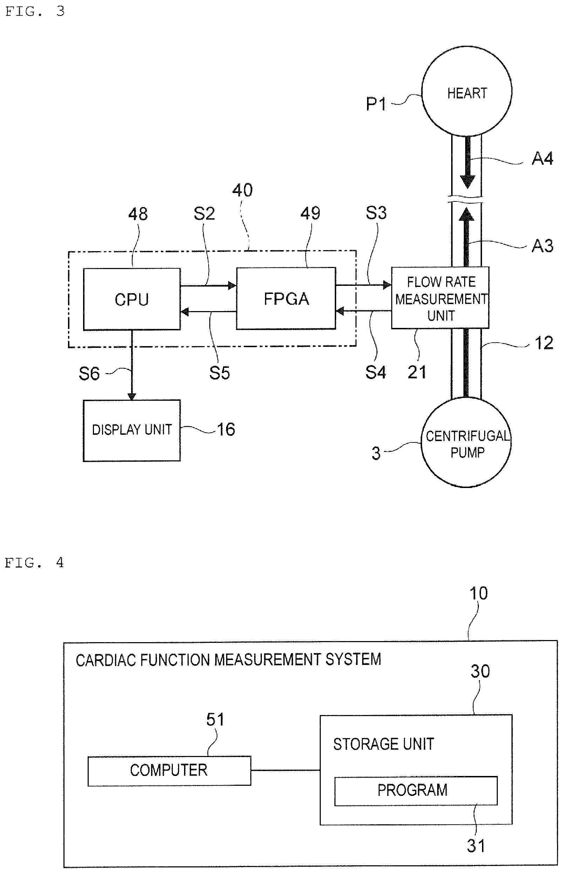

Fig. 3 is a block diagram illustrating the control unit and the flow rate measurement unit according to the present embodiment.

In the block diagram shown in Fig. 3, the oxygenator is omitted for convenience of explanation.

As indicated by an arrow A1 shown in Fig. 2 and an arrow A3 shown in Fig. 3, the blood returned from the centrifugal pump 3 to the patient P via the blood transfer tube 12 and the blood transfer side catheter 6 flows toward a heart P1 of the patient P through, for example, the artery. On the other hand, as indicated by an arrow A2 shown in Fig. 2 and an arrow A4 shown in Fig. 3, the blood transferred from the heart P1 of the patient P and oxygenated in the lungs of the patient P passes through the artery and flows toward peripheral blood vessels of the head and peripheral blood vessels of the lower limb.

As described above, a direction of the flow of the blood returned into the body of the patient P by the centrifugal pump 3 is opposite to a direction of the flow of the blood flowing through the inside of the body of the patient P. That is, the centrifugal pump 3 returns the blood that has passed through the oxygenator 2 to the patient P in a state in which the blood flows retrogradely.

As shown in Fig. 3, the control unit 40 of the cardiac function measurement system 10 includes a central processing unit (CPU) 48 and a field programmable gate array (FPGA) 49. For example, the CPU 48 transmits, to the FPGA 49 every 50 milliseconds (ms), a signal S2 for requesting a measurement of the flow rate of the blood transferred from the centrifugal pump 3. The FPGA 49 receives the signal S2 transmitted from the CPU 48, and transmits, to the flow rate measurement unit 21 every 50 ms, a signal S3 for requesting the measurement of the flow rate of the blood transferred from the centrifugal pump 3. The flow rate measurement unit 21 receives the signal S3 transmitted from the FPGA 49, measures, every 50 ms, the flow rate of the blood transferred from the centrifugal pump 3, and transmits a signal S4 related to the measured flow rate of the blood to the FPGA 49. The FPGA 49 receives the signal S4 transmitted from the flow rate measurement unit 21, and transmits, to the CPU 48, a signal S5 related to an average value of the flow rate in a most recent one second.

In the present embodiment, the flow rate measurement unit 21 measures the flow rate of the blood every 50 ms and transmits the signal S4 related to the measured flow rate of the blood to the FPGA 49. Therefore, 20 pieces (1 s/50 ms) of measurement data are present per second. Therefore, the FPGA 49 calculates an average value of the 20 pieces of measurement data as the average value of the flow rate in the most recent one second, and transmits the signal S5 related to the average value of the flow rate to the CPU 48. The CPU 48 receives the signal S5 transmitted from the FPGA 49, and performs control to display the flow rate of the blood transferred from the centrifugal pump 3 on the external monitor (display unit) 16 as the measured blood transfer flow rate. In the present embodiment, the measured blood transfer flow rate is the average value of the flow rates in the most recent one second, that is, the average value of the 20 pieces of measurement data.

A period in which the flow rate measurement unit 21 measures the flow rate of the blood is not limited to 50 ms. A predetermined time for the CPU 48 to calculate the average value of the flow rate of the blood is not limited to one second.

Fig. 4 is a block diagram illustrating a configuration of main units of the cardiac function measurement system according to the present embodiment.

The cardiac function measurement system 10 according to the present embodiment includes a computer 51 and a storage unit 30. The computer 51 includes the control unit 40 (see Figs. 1 and 5), reads a program 31 stored in the storage unit 30, and executes various calculations and processes. The storage unit 30 stores the program 31 (cardiac function measurement program) executed by the computer 51. The program 31 according to the present embodiment is an example of the "cardiac function measurement program" according to this disclosure. Examples of the storage unit 30 include a hard disk drive (HDD). The program 31 is not limited to being stored in the storage unit 30, and may be stored in advance and distributed in a computer-readable storage medium, or may be downloaded to the cardiac function measurement system 10 via a network. The storage unit 30 may be an external storage device connected to the computer 51.

Next, the configuration of the main units of the cardiac function measurement system 10 according to the present embodiment will be further described with reference to the drawings.

Fig. 5 is a block diagram illustrating the configuration of the main units of the cardiac function measurement system according to the present embodiment.

The cardiac function measurement system 10 according to the present embodiment includes the control unit 40, the storage unit 30, the touch panel 52, and a communication unit 53. The control unit 40 reads the program 31 (see Fig. 4) stored in the storage unit 30 and executes various calculations and processes. The control unit 40 includes a display processing unit 41, a notification processing unit 42, a pump discharge pressure determination unit 43, a withdrawal reference flow rate determination unit 44, a measured blood transfer flow rate determination unit 45, a parameter calculation unit 46, and a withdrawal timing determination unit 47. The display processing unit 41, the notification processing unit 42, the pump discharge pressure determination unit 43, the withdrawal reference flow rate determination unit 44, the measured blood transfer flow rate determination unit 45, the parameter calculation unit 46, and the withdrawal timing determination unit 47 are implemented by the computer 51 executing the program 31 stored in the storage unit 30. The display processing unit 41, the notification processing unit 42, the pump discharge pressure determination unit 43, the withdrawal reference flow rate determination unit 44, the measured blood transfer flow rate determination unit 45, the parameter calculation unit 46, and the withdrawal timing determination unit 47 may be implemented by hardware or may be implemented by a combination of hardware and software. The storage unit 30 stores the program 31 described above with reference to Fig. 4, and includes a withdrawal reference flow rate storage unit 32 and a pump characteristic storage unit 33.

The display processing unit 41 executes a process of displaying, on the external monitor 16, at least one of the rotation speed of the centrifugal pump 3 based on the signal G that is related to the rotation speed and that is transmitted from the centrifugal pump 3, a withdrawal reference flow rate determined by the withdrawal reference flow rate determination unit 44, the measured blood transfer flow rate determined by the measured blood transfer flow rate determination unit 45, a parameter calculated by the parameter calculation unit 46, and a withdrawal timing of the extracorporeal circulator 1 determined by the withdrawal timing determination unit 47. In the present embodiment, the "withdrawal reference flow rate" is a theoretical flow rate of the blood transferred from the centrifugal pump 3 based on a discharge pressure of the centrifugal pump 3 when the heart of the patient P is healthy and strong, and is determined by the withdrawal reference flow rate determination unit 44. The "measured blood transfer flow rate" is a realistic flow rate of the blood (in the present embodiment, the blood transferred from the centrifugal pump 3) flowing through the circulation circuit 1R, and is determined by the measured blood transfer flow rate determination unit 45 based on a measurement result of the flow rate measurement unit 21. Details of the parameter calculated by the parameter calculation unit 46 and the withdrawal timing of the extracorporeal circulator 1 will be described later.

As will be described later, the display processing unit 41 can display a graph representing a relationship between the rotation speed of the centrifugal pump 3 and the measured blood transfer flow rate on the external monitor 16, and can display a graph representing a relationship between the parameter calculated by the parameter calculation unit 46 and an elapsing time on the external monitor 16.

When a predetermined condition is satisfied, the notification processing unit 42 executes a process of displaying on the external monitor 16 that the withdrawal timing of the extracorporeal circulator 1 is appropriate or displaying on the external monitor 16 that the withdrawal timing of the extracorporeal circulator 1 is late. The notification processing unit 42 may execute a notification method by, for example, generating light or sound.

The pump discharge pressure determination unit 43 refers to the pump characteristic of the centrifugal pump 3 stored in the pump characteristic storage unit 33, and determines the discharge pressure of the centrifugal pump 3 based on the signal G (that is, the rotation speed of the centrifugal pump 3) related to the rotation speed transmitted from the centrifugal pump 3.

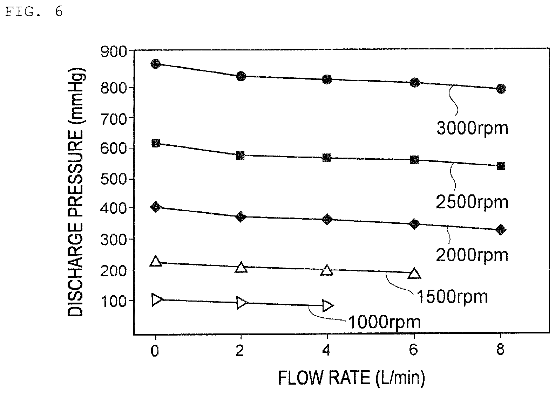

Here, an example of the pump characteristic of the centrifugal pump 3 stored in the pump characteristic storage unit 33 will be described with reference to Fig. 6.

Fig. 6 is a graph showing the example of the pump characteristic stored in the pump characteristic storage unit according to the present embodiment.

A horizontal axis of the graph shown in Fig. 6 represents the flow rate (L/min) of the blood transferred from the centrifugal pump 3. A vertical axis of the graph shown in Fig. 6 represents the discharge pressure (mmHg) of the centrifugal pump 3. As shown in Fig. 6, the relationship between the flow rate in the centrifugal pump 3 and the discharge pressure of the centrifugal pump 3 is represented by a curve or a straight line for each rotation speed of the centrifugal pump 3. The discharge pressure of the centrifugal pump 3 slightly decreases as the flow rate in the centrifugal pump 3 increases, and when the rotational speed of the centrifugal pump 3 is the same, the discharge pressure of the centrifugal pump 3 does not change much and is substantially constant depending on the flow rate in the centrifugal pump 3.

For example, when the rotation speed of the centrifugal pump 3 is 1000 rpm and the flow rate in the centrifugal pump 3 is 2 L/min, the discharge pressure of the centrifugal pump 3 is approximately 100 mmHg. For example, when the rotation speed of the centrifugal pump 3 is 2000 rpm and the flow rate in the centrifugal pump 3 is 2 L/min, the discharge pressure of the centrifugal pump 3 is approximately 400 mmHg. For example, when the rotation speed of the centrifugal pump 3 is 3000 rpm and the flow rate in the centrifugal pump 3 is 2 L/min, the discharge pressure of the centrifugal pump 3 is approximately 900 mmHg. As described above, in the example of the pump characteristic shown in Fig. 6, when the rotation speed of the centrifugal pump 3 is doubled, the discharge pressure of the centrifugal pump 3 becomes approximately four times, and when the rotation speed of the centrifugal pump 3 is tripled, the discharge pressure of the centrifugal pump 3 becomes approximately nine times. That is, in the example of the pump characteristic shown in Fig. 6, the discharge pressure of the centrifugal pump 3 is substantially proportional to a square of the rotation speed of the centrifugal pump 3.

In this manner, the pump discharge pressure determination unit 43 refers to the pump characteristic of the centrifugal pump 3 stored in the pump characteristic storage unit 33, and determines the discharge pressure of the centrifugal pump 3 based on the rotation speed of the centrifugal pump 3.

The pump characteristic of the centrifugal pump 3 stored in the pump characteristic storage unit 33 is not limited to the graph shown in Fig. 6, and may be other graphs. In that case, as the pump characteristic of the centrifugal pump, depending on a type of the centrifugal pump, there is a centrifugal pump in which a change in the discharge pressure with respect to a change in the flow rate is relatively large. Therefore, when such a centrifugal pump 3 is used, the pump discharge pressure determination unit 43 can determine the discharge pressure of the centrifugal pump 3 based on, in addition to the signal G that is related to the rotation speed and that is transmitted from the centrifugal pump 3, the signal S4 transmitted from the flow rate measurement unit 21 or the measured blood transfer flow rate determined by the measured blood transfer flow rate determination unit 45. The pump characteristic of the centrifugal pump 3 stored in the pump characteristic storage unit 33 is not limited to the graph representing the relationship between the rotation speed of the centrifugal pump 3 and the discharge pressure of the centrifugal pump 3, and may be, for example, a numerical formula representing the relationship between the rotation speed of the centrifugal pump 3 and the discharge pressure of the centrifugal pump 3, or a form (table) representing the relationship between the rotation speed of the centrifugal pump 3 and the discharge pressure of the centrifugal pump 3.

Next, returning to Fig. 5, the configuration of the main units of the cardiac function measurement system 10 according to the present embodiment will be further described.

The withdrawal reference flow rate determination unit 44 refers to a withdrawal reference flow rate characteristic stored in the withdrawal reference flow rate storage unit 32, and determines the withdrawal reference flow rate indicating the theoretical flow rate of the blood transferred from the centrifugal pump 3 based on the discharge pressure of the centrifugal pump 3 determined by the pump discharge pressure determination unit 43. The withdrawal reference flow rate characteristic stored in the withdrawal reference flow rate storage unit 32 represents a relationship between the discharge pressure or the rotation speed of the centrifugal pump 3 and the flow rate in the centrifugal pump 3 when the heart of the patient P is healthy and strong. For example, the withdrawal reference flow rate characteristic stored in the withdrawal reference flow rate storage unit 32 is a graph, a numerical formula, a form (table), or the like representing the relationship between the discharge pressure or the rotation speed of the centrifugal pump 3 and the flow rate in the centrifugal pump 3 when the heart of the patient P is healthy and strong.

The withdrawal reference flow rate characteristic stored in the withdrawal reference flow rate storage unit 32 may include patient information (height, weight, age, gender, and the like). In this case, in consideration of the patient information, the withdrawal reference flow rate determination unit 44 can determine the withdrawal reference flow rate based on the discharge pressure of the centrifugal pump 3 determined by the pump discharge pressure determination unit 43.

The measured blood transfer flow rate determination unit 45 determines the realistic flow rate of the blood (in the present embodiment, the blood transferred from the centrifugal pump 3) flowing through the circulation circuit 1R based on the measurement result of the flow rate measurement unit 21. The measured blood transfer flow rate determined by the measured blood transfer flow rate determination unit 45 is an average flow rate indicating an average value of a plurality of realistic flow rates measured by the flow rate measurement unit 21 in a predetermined time. For example, as described above with reference to Figs. 2 and 3, the measured blood transfer flow rate determination unit 45 calculates the average value of 20 pieces of measurement data as the average value of the flow rate in the most recent one second, and determines the average flow rate indicated by the average value as the measured blood transfer flow rate.

As described above with reference to Figs. 2 and 3, the direction of the flow of the blood returned into the body of the patient P by the centrifugal pump 3 is opposite to the direction of the flow of the blood flowing through the body of the patient P. Therefore, when the heart of the patient P is healthy and strong, the blood returned into the body of the patient P by the centrifugal pump 3 tends to be relatively strongly pushed back by the blood transferred from the heart P1 of the patient P. Accordingly, when the heart of the patient P is healthy and strong, the flow rate of the blood transferred from the centrifugal pump 3 is relatively small. In other words, when the heart of the patient P is healthy and strong, the flow rate of the blood measured by the flow rate measurement unit 21 is relatively small.

On the other hand, when the heart of the patient P is not healthy and is weak, the blood returned into the body of the patient P by the centrifugal pump 3 tends to be relatively weakly pushed back by the blood transferred from the heart P1 of the patient P. Accordingly, when the heart of the patient P is not healthy and is weak, the flow rate of the blood transferred from the centrifugal pump 3 is relatively large. In other words, when the heart of the patient P is not healthy and is weak, the flow rate of the blood measured by the flow rate measurement unit 21 is relatively large.

The parameter calculation unit 46 calculates a parameter indicating a comparison between the withdrawal reference flow rate determined by the withdrawal reference flow rate determination unit 44 and the measured blood transfer flow rate determined by the measured blood transfer flow rate determination unit 45. Specifically, the parameter calculation unit 46 calculates a difference parameter representing a difference between the withdrawal reference flow rate determined by the withdrawal reference flow rate determination unit 44 and the measured blood transfer flow rate determined by the measured blood transfer flow rate determination unit 45. Details of the parameter calculated by the parameter calculation unit 46 will be described later.

The withdrawal timing determination unit 47 determines the withdrawal timing of the extracorporeal circulator 1 based on the comparison between the withdrawal reference flow rate and the measured blood transfer flow rate. Specifically, the withdrawal timing determination unit 47 determines the withdrawal timing of the extracorporeal circulator 1 based on the parameter calculated by the parameter calculation unit 46. For example, when the parameter calculated by the parameter calculation unit 46 is included in a predetermined range, the withdrawal timing determination unit 47 determines that the withdrawal timing of the extracorporeal circulator 1 is appropriate. On the other hand, when the parameter calculated by the parameter calculation unit 46 is not included in the predetermined range, the withdrawal timing determination unit 47 determines that the withdrawal timing of the extracorporeal circulator 1 is not appropriate.

The withdrawal reference flow rate storage unit 32 stores the withdrawal reference flow rate characteristic. The withdrawal reference flow rate characteristic represents the relationship between the discharge pressure or the rotation speed of the centrifugal pump 3 and the flow rate in the centrifugal pump 3 when the heart of the patient P is healthy and strong. For example, the withdrawal reference flow rate characteristic is a graph, a numerical formula, a form (table), or the like representing the relationship between the discharge pressure or the rotation speed of the centrifugal pump 3 and the flow rate in the centrifugal pump 3 when the heart of the patient P is healthy and strong. The withdrawal reference flow rate characteristic may include the patient information (height, weight, age, gender, and the like).

The pump characteristic storage unit 33 stores a characteristic of the centrifugal pump 3. For example, as shown in Fig. 6, the characteristic of the centrifugal pump 3 shows the relationship between the flow rate (L/min) of the blood transferred from the centrifugal pump 3 and the discharge pressure (that is, a lifting height) (mmHg) of the centrifugal pump 3. For example, in the graph showing the characteristic of the centrifugal pump 3, the relationship between the flow rate (L/min) of the blood transferred from the centrifugal pump 3 and the discharge pressure (mmHg) of the centrifugal pump 3 is set according to the rotation speed (rpm) of the centrifugal pump 3.

The touch panel 52 is an example of the "display unit" according to this disclosure, and can display various types of information and detect contact, or the like, of a finger of an operator or the like. The touch panel 52 transmits, to the control unit 40, information input by an operator or the like in response to an operation of the operator or the like.

The communication unit 53 communicates with the drive motor 4, the external monitor 16, and the flow rate measurement unit 21, and transmits and receives various types of information and various types of signals.

Here, for the auxiliary circulation operation using the extracorporeal circulator 1, one of important factors is to determine whether the cardiac function (state of the heart P1) of the patient P is recovered. That is, when the withdrawal timing of the extracorporeal circulator 1 is too early with respect to the recovery of the cardiac function of the patient P, the heart P1 of the patient P cannot stand the withdrawal timing, and it may be necessary to reattach the extracorporeal circulator 1 or the patient P may die. On the other hand, when the withdrawal timing of the extracorporeal circulator 1 is too late with respect to the recovery of the cardiac function of the patient P, complications such as bleeding may occur due to anticoagulant therapy, consumption of a blood plasma component, or the like. That is, there is an appropriate timing for the withdrawal of the extracorporeal circulator 1. Further, it is desirable that the withdrawal timing of the extracorporeal circulator 1 can be early ascertained.

As described above with reference to Figs. 2 and 3, the direction of the flow of the blood returned into the body of the patient P by the centrifugal pump 3 is opposite to the direction of the flow of the blood flowing through the body of the patient P. That is, the centrifugal pump 3 returns the blood that has passed through the oxygenator 2 to the patient P in a state in which the blood flows retrogradely. Therefore, it is difficult to only measure a cardiac output, and it is difficult to determine recovery of the cardiac function of a patient with high accuracy.

On the other hand, in the cardiac function measurement system 10 according to the present invention, the withdrawal reference flow rate determination unit 44 determines the withdrawal reference flow rate indicating the theoretical flow rate of the blood transferred from the centrifugal pump 3 based on the discharge pressure of the centrifugal pump 3 determined by the pump discharge pressure determination unit 43 based on the rotation speed of the centrifugal pump 3. The measured blood transfer flow rate determination unit 45 determines the measured blood transfer flow rate indicating the realistic flow rate of the blood transferred from the centrifugal pump 3 based on the measurement result of the flow rate measurement unit 21 that measures the flow rate of the blood transferred from the centrifugal pump 3. For example, the measured blood transfer flow rate determination unit 45 determines, as the measured blood transfer flow rate, the average flow rate indicating the average value of the plurality of realistic flow rates measured by the flow rate measurement unit 21 in the predetermined time. The parameter calculation unit 46 calculates the parameter indicating the comparison between the withdrawal reference flow rate determined by the withdrawal reference flow rate determination unit 44 and the measured blood transfer flow rate determined by the measured blood transfer flow rate determination unit 45. Then, the withdrawal timing determination unit 47 determines the withdrawal timing of the extracorporeal circulator 1 based on the comparison between the withdrawal reference flow rate and the measured blood transfer flow rate. Specifically, the withdrawal timing determination unit 47 determines the withdrawal timing of the extracorporeal circulator 1 based on the parameter calculated by the parameter calculation unit 46. That is, the withdrawal timing determination unit 47 determines whether the withdrawal timing of the extracorporeal circulator 1 is appropriate.

According to the present embodiment, the cardiac function measurement system 10 compares the withdrawal reference flow rate with the measured blood transfer flow rate having a relatively small temporal change, and determines the withdrawal timing of the extracorporeal circulator 1. Accordingly, the cardiac function measurement system 10 according to the present embodiment can determine the recovery of the cardiac function of the patient P with high accuracy. When the withdrawal timing determination unit 47 determines the withdrawal timing of the extracorporeal circulator 1 based on the parameter calculated by the parameter calculation unit 46, the cardiac function measurement system 10 can quantitatively measure output ability of the patient P and determine the withdrawal timing of the extracorporeal circulator 1 based on a quantitative comparison. Accordingly, the cardiac function measurement system 10 according to the present embodiment can determine the recovery of the cardiac function of the patient P with high accuracy.

Next, a specific example of the operations of the cardiac function measurement system according to the present embodiment will be described with reference to the drawings.

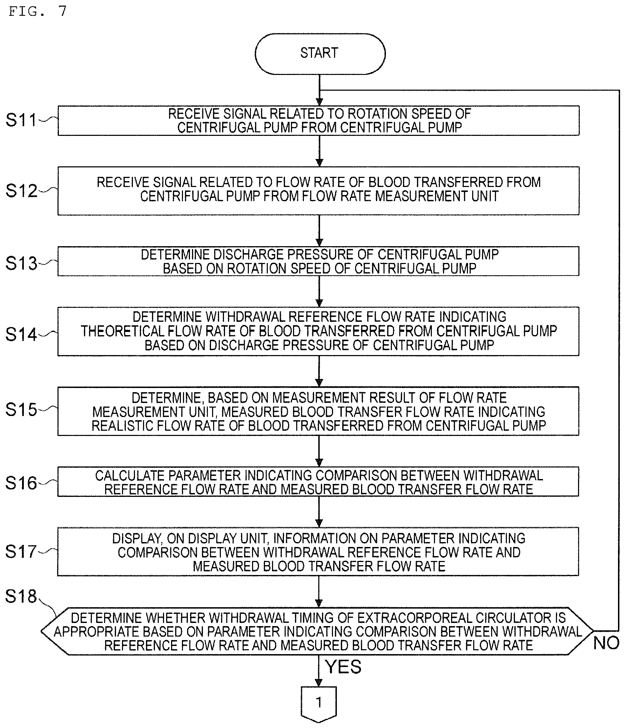

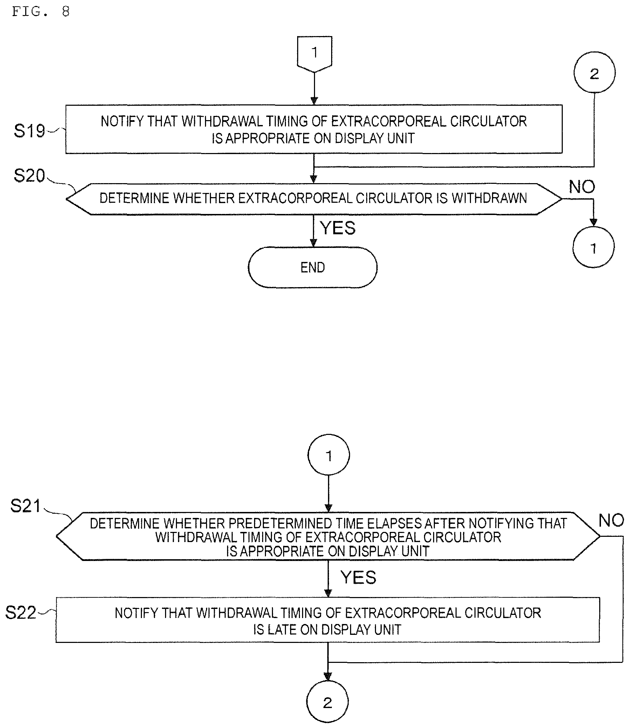

Figs. 7 and 8 are flowcharts showing the specific example of the operations of the cardiac function measurement system according to the present embodiment.

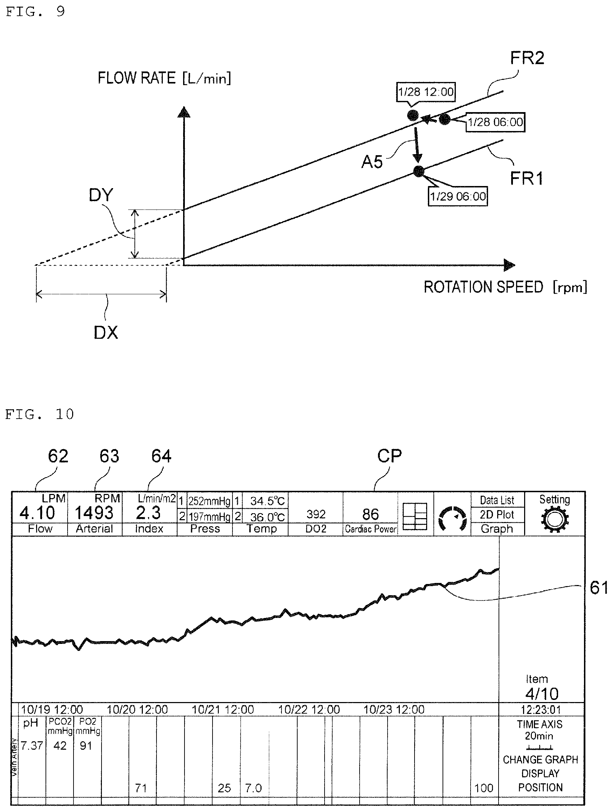

Fig. 9 is a schematic view showing a first specific example of a screen displayed on the display unit according to the present embodiment.

Fig. 10 is a schematic view showing a second specific example of the screen displayed on the display unit according to the present embodiment.

The specific example of the operations of the cardiac function measurement system 10 according to the present embodiment will be described with reference to Figs. 7 and 8. In the present specific example, for convenience of explanation, a process executed after the extracorporeal circulator 1 is attached to the patient P will be described.

First, in step S11, the cardiac function measurement system 10 receives the signal G (see Fig. 1) related to the rotation speed of the centrifugal pump 3 from the centrifugal pump 3. Specifically, the communication unit 53 of the cardiac function measurement system 10 receives the signal G related to the rotation speed of the centrifugal pump 3 from the drive motor 4 that drives the centrifugal pump 3.

Subsequently, in step S12, the cardiac function measurement system 10 receives, from the flow rate measurement unit 21 via the communication unit 53, the signal S1 (see Fig. 1) related to the flow rate of the blood transferred from the centrifugal pump 3. The process in step S12 may not necessarily be executed after the process in step S11, and may be executed simultaneously with the process in step S11.

Subsequently, in step S13, the pump discharge pressure determination unit 43 refers to the pump characteristic of the centrifugal pump 3 stored in the pump characteristic storage unit 33, and determines the discharge pressure of the centrifugal pump 3 based on the rotation speed of the centrifugal pump 3. The pump characteristic of the centrifugal pump 3 stored in the pump characteristic storage unit 33 is as described above with reference to Figs. 5 and 6.

Subsequently, in step S14, the withdrawal reference flow rate determination unit 44 refers to the withdrawal reference flow rate characteristic stored in the withdrawal reference flow rate storage unit 32, and determines the withdrawal reference flow rate indicating the theoretical flow rate of the blood transferred from the centrifugal pump 3 based on the discharge pressure of the centrifugal pump 3 determined by the pump discharge pressure determination unit 43.

Subsequently, in step S15, the measured blood transfer flow rate determination unit 45 determines the realistic flow rate of the blood transferred from the centrifugal pump 3 based on the measurement result of the flow rate measurement unit 21. For example, as described above with reference to Figs. 2 and 3, the measured blood transfer flow rate determination unit 45 calculates the average value of 20 pieces of measurement data as the average value of the flow rate in the most recent one second, and determines the average flow rate indicated by the average value as the measured blood transfer flow rate. That is, for example, the measured blood transfer flow rate determined by the measured blood transfer flow rate determination unit 45 is the average flow rate indicating the average value of the plurality of realistic flow rates measured by the flow rate measurement unit 21 in the predetermined time.

Subsequently, in step S16, the parameter calculation unit 46 calculates the parameter indicating the comparison between the withdrawal reference flow rate determined by the withdrawal reference flow rate determination unit 44 and the measured blood transfer flow rate determined by the measured blood transfer flow rate determination unit 45.

The parameter calculation unit 46 calculates the difference parameter representing the difference between the withdrawal reference flow rate determined by the withdrawal reference flow rate determination unit 44 and the measured blood transfer flow rate determined by the measured blood transfer flow rate determination unit 45.

Subsequently, in step S17, the display processing unit 41 executes a process of displaying, on the external monitor (display unit) 16, information on the parameter that is calculated by the parameter calculation unit 46 and that indicates the comparison between the withdrawal reference flow rate and the measured blood transfer flow rate.

Here, the first specific example of the screen displayed on the external monitor 16 will be described with reference to Fig. 9. In the first specific example shown in Fig. 9, the display processing unit 41 displays, on the external monitor 16, a graph representing a relationship between the rotation speed of the centrifugal pump 3 and the withdrawal reference flow rate and the measured blood transfer flow rate. A horizontal axis of the graph shown in Fig. 9 represents the rotation speed (rpm) of the centrifugal pump 3. A vertical axis of the graph shown in Fig. 9 represents the flow rate (L/min) of the blood transferred from the centrifugal pump 3.

A line FR1 shown in Fig. 9 is an approximate line related to the withdrawal reference flow rate characteristic. In other words, the line FR1 shown in Fig. 9 is an approximate line representing the relationship between the rotation speed of the centrifugal pump 3 and the flow rate in the centrifugal pump 3 when the heart of the patient P is healthy and strong. The approximate line related to the withdrawal reference flow rate characteristic is not limited to the regression line (line FR1) shown in Fig. 9, and may be a quadratic curve. For example, the line FR1 shown in Fig. 9 is displayed in a bluish color such as blue or green on the external monitor 16.

A line FR2 shown in Fig. 9 is an approximate line related to the measured blood transfer flow rate determined by the measured blood transfer flow rate determination unit 45 when the heart of the patient P is not healthy and is weak. In other words, the line FR2 is an approximate line representing the relationship between the rotation speed of the centrifugal pump 3 and the flow rate in the centrifugal pump 3 when the heart of the patient P is not healthy and is weak. The approximate line related to the measured blood transfer flow rate is not limited to the regression line (line FR2) shown in Fig. 9, and may be a quadratic curve. For example, the line FR2 shown in Fig. 9 is displayed in a reddish color such as red, pink, or orange on the external monitor 16.

A slope of the regression line (line FR1 and line FR2) shown in Fig. 9 is, for example, approximately 0.0071 (L/min/rpm). However, the slope of the regression line (line FR1 and line FR2) is not limited to 0.0071 (L/min/rpm). The slope of the regression line (line FR1 and line FR2) may be calculated based on "an amount of a change in the flow rate in the centrifugal pump 3/an amount of a change in the rotation speed of the centrifugal pump 3" by the operator or the like changing, in order to match arrangement conditions of the case, the rotation speed of the centrifugal pump 3 by, for example, approximately ±300 rpm in an initial stage in which the extracorporeal circulator 1 is attached to the patient P.

In the first specific example shown in Fig. 9, marks at "6:00 on January 28" and "12:00 on January 28" representing the relationship between the rotation speed of the centrifugal pump 3 and the measured blood transfer flow rate are located near the line FR2. On the other hand, a mark at "6:00 on January 29" representing the relationship between the rotation speed of the centrifugal pump 3 and the measured blood transfer flow rate is located near the line FR1. As described above, when the cardiac function of the patient P is recovered, the blood returned into the body of the patient P by the centrifugal pump 3 relatively strongly tends to be pushed back by the blood transferred from the heart P1 of the patient P, and as compared with the flow rate at the same rotation speed of the centrifugal pump 3, the measured blood transfer flow rate decreases. That is, when the cardiac function of the patient P is recovered, as indicated by an arrow A5 shown in Fig. 9, the mark representing the relationship between the rotation speed of the centrifugal pump 3 and the measured blood transfer flow rate moves downward and approaches the line FR1 serving as the approximate line related to the withdrawal reference flow rate characteristic.

In the first specific example shown in Fig. 9, the parameter calculated by the parameter calculation unit 46 is displayed on the external monitor 16 as a difference parameter representing a difference between the withdrawal reference flow rate and the measured blood transfer flow rate at the same rotation speed of the centrifugal pump 3. That is, in the first specific example shown in Fig. 9, the parameter calculated by the parameter calculation unit 46 corresponds to a difference DY between an intercept of a flow rate axis (Y axis) of the line FR2 and an intercept of the flow rate axis (Y axis) of the line FR1.

Alternatively, the parameter calculated by the parameter calculation unit 46 may be displayed on the external monitor 16 as a difference parameter representing a difference between the withdrawal reference flow rate and the measured blood transfer flow rate at the same flow rate of the blood transferred from the centrifugal pump 3. That is, the parameter calculated by the parameter calculation unit 46 may correspond to a difference DX between an intercept of a rotation speed axis (X axis) of the line FR2 and an intercept of the rotation speed axis (X axis) of the line FR1.

The display processing unit 41 may display, between the line FR1 and the line FR2, a plurality of approximate lines representing the relationship between the rotation speed of the centrifugal pump 3 and the flow rate in the centrifugal pump 3. That is, the display processing unit 41 may display, between the line FR1 and the line FR2, a plurality of approximate lines indicating degrees of the recovery of the cardiac function of the patient P. Alternatively, the display processing unit 41 may display, between the line FR1 and the line FR2, the degrees of the recovery of the cardiac function of the patient P in colors or a shading gradation.

The second specific example of the screen displayed on the external monitor 16 will be described with reference to Fig. 10. In the second specific example shown in Fig. 10, the display processing unit 41 displays, on the external monitor 16, the graph representing the relationship between the parameter calculated by the parameter calculation unit 46 and the elapsing time, and the parameter calculated by the parameter calculation unit 46. That is, the second specific example shown in Fig. 10 displays a graph 61 representing the relationship between the parameter calculated by the parameter calculation unit 46 and the elapsing time. A horizontal axis of the graph 61 shown in Fig. 10 represents the elapsing time (for example, date and time) after the extracorporeal circulator 1 is attached to the patient P. A vertical axis of the graph 61 shown in Fig. 10 represents the parameter calculated by the parameter calculation unit 46.

In the second specific example shown in Fig. 10, the parameter calculated by the parameter calculation unit 46 is calculated using, for example, the following equation, and is displayed on the external monitor 16 as the difference parameter representing the difference between the withdrawal reference flow rate and the measured blood transfer flow rate at the same rotation speed of the centrifugal pump 3.

$$\text{Parameter CP} = (0.0071 \times M - F - 2.7) \times 90 \ \ldots \ \text{Equation}$$

$$(1)$$

As described above with reference to Fig. 9, a parameter CP corresponds to the difference DY between the intercept of the flow rate axis (Y axis) of the line FR2 and the intercept of the flow rate axis (Y axis) of the line FR1. The symbol "M" in Equation (1) represents the rotation speed of the centrifugal pump 3 received from the centrifugal pump 3 by the communication unit 53. The symbol "F" in Equation (1) represents the measured blood transfer flow rate determined by the measured blood transfer flow rate determination unit 45.

The numerical value "0.0071" in Equation (1) corresponds to the slope of the regression line (line FR1 and line FR2) described above with reference to Fig. 9. As described above with reference to Fig. 9, the slope of the regression line (line FR1 and line FR2), that is, the numerical value "0.0071" in Equation (1) is not limited to 0.0071 (L/min/rpm). The numerical values "2.7" and "90" in Equation (1) are numerical values for convenience in easily grasping or identifying the parameter CP, and may be appropriately set in consideration of a "weight" or the like.

In the second specific example shown in Fig. 10, when the cardiac function of the patient P is recovered, and the blood returned into the body of the patient P by the centrifugal pump 3 relatively strongly tends to be pushed back by the blood transferred from the heart P1 of the patient P, the parameter CP calculated using Equation (1) is set to be large. That is, the graph 61 shown in Fig. 10 shows a state in which the cardiac function of the patient P is recovered with the elapsing time.

In the second specific example shown in Fig. 10, the parameter CP calculated by the parameter calculation unit 46 is displayed as a numerical value on the external monitor 16. Accordingly, the operator or the like can intuitively grasp the parameter CP indicating the degree of the recovery of the cardiac function of the patient P as the numerical value by checking the external monitor 16.

In the second specific example shown in Fig. 10, a measured blood transfer flow rate (LMP: L/min) 62 determined by the measured blood transfer flow rate determination unit 45, a rotation speed (RPM) 63 of the centrifugal pump 3 received from the centrifugal pump 3 by the communication unit 53, and an Index (L/min/m2) 64 indicating a flow rate of blood per body surface of the patient P are displayed on the external monitor 16.

Returning to Fig. 7, the specific example of the operations of the cardiac function measurement system 10 according to the present embodiment will be further described. In step S18 following step S17, the withdrawal timing determination unit 47 determines the withdrawal timing of the extracorporeal circulator 1 based on the parameter that is calculated by the parameter calculation unit 46 and that indicates the comparison between the withdrawal reference flow rate and the measured blood transfer flow rate. That is, the withdrawal timing determination unit 47 determines whether the withdrawal timing of the extracorporeal circulator 1 is appropriate. For example, as indicated by the arrow A5 shown in Fig. 9, when the mark (circular mark in Fig. 9) representing the relationship between the rotation speed of the centrifugal pump 3 and the measured blood transfer flow rate approaches the line FR1 serving as the approximate line related to the withdrawal reference flow rate characteristic and the parameter calculated by the parameter calculation unit 46 is included in the predetermined range, the withdrawal timing determination unit 47 determines that the withdrawal timing of the extracorporeal circulator 1 is appropriate. For example, in the first specific example described above with reference to Fig. 9, when the difference DY between the intercept of the flow rate axis (Y axis) of the line FR2 and the intercept of the flow rate axis (Y axis) of the line FR1 is equal to or less than a predetermined value, the withdrawal timing determination unit 47 determines that the withdrawal timing of the extracorporeal circulator 1 is appropriate. For example, in the second specific example described above with reference to Fig. 10, when the parameter CP calculated by the parameter calculation unit 46 is equal to or more than a predetermined value, the withdrawal timing determination unit 47 determines that the withdrawal timing of the extracorporeal circulator 1 is appropriate.

According to the invention, when the withdrawal timing of the extracorporeal circulator 1 is appropriate (step S18: YES), in step S19, the notification processing unit 42 notifies on the external monitor 16 that the withdrawal timing of the extracorporeal circulator 1 is appropriate. For example, in the first specific example described above with reference to Fig. 9, when the withdrawal timing of the extracorporeal circulator 1 is appropriate, the notification processing unit 42 adds a bluish color such as blue or green to the mark (circular mark in Fig. 9) representing the relationship between the rotation speed of the centrifugal pump 3 and the measured blood transfer flow rate. For example, in the second specific example described above with reference to Fig. 10, when the withdrawal timing of the extracorporeal circulator 1 is appropriate, the notification processing unit 42 adds a bluish color such as blue or green to the graph 61 representing the relationship between the parameter calculated by the parameter calculation unit 46 and the elapsing time, and the parameter CP calculated by the parameter calculation unit 46.

Alternatively, when the withdrawal timing of the extracorporeal circulator 1 is appropriate, the notification processing unit 42 may display on the external monitor 16 that the withdrawal timing of the extracorporeal circulator 1 is appropriate in a pop-up manner using characters, figures, symbols, or the like. Alternatively, the notification processing unit 42 may notify, by a sound, that the withdrawal timing of the extracorporeal circulator 1 is appropriate.

On the other hand, when the withdrawal timing of the extracorporeal circulator 1 is not appropriate (step S18: NO), the process described above in step S11 is executed. In step S20 following step S19, the control unit 40 determines whether the extracorporeal circulator 1 is withdrawn from the patient P. When the extracorporeal circulator 1 is withdrawn from the patient P (step S20: YES), the control unit 40 ends the operations of the cardiac function measurement system 10.

On the other hand, when the extracorporeal circulator 1 is not withdrawn from the patient P (step S20: NO), in step S21, the control unit 40 determines whether a predetermined time elapses after the notification processing unit 42 notifies on the external monitor 16 that the withdrawal timing of the extracorporeal circulator 1 is appropriate. For example, the control unit 40 uses a timer function of the control unit 40 to measure an elapsing time after the notification processing unit 42 notifies on the external monitor 16 that the withdrawal timing of the extracorporeal circulator 1 is appropriate.

When the predetermined time elapses after the notification processing unit 42 notifies on the external monitor 16 that the withdrawal timing of the extracorporeal circulator 1 is appropriate (step S21: YES), in step S22, the notification processing unit 42 notifies on the external monitor 16 that the timing at which the extracorporeal circulator 1 is withdrawn from the patient P is late. For example, the notification processing unit 42 displays on the external monitor 16 that the timing at which the extracorporeal circulator 1 is withdrawn from the patient P is late in a pop-up manner using characters, figures, symbols, or the like. Alternatively, the notification processing unit 42 may notify, by a sound, that the timing at which the extracorporeal circulator 1 is withdrawn from the patient P is late.

On the other hand, when the predetermined time does not elapse after the notification processing unit 42 notifies on the external monitor 16 that the withdrawal timing of the extracorporeal circulator 1 is appropriate (step S21: NO), the process described above with reference to step S20 is executed.

According to the cardiac function measurement system 10 in the present specific example, the parameter calculation unit 46 can calculate the difference parameter representing the difference between the withdrawal reference flow rate and the measured blood transfer flow rate as the parameter indicating the comparison between the withdrawal reference flow rate and the measured blood transfer flow rate using a relatively simple numerical formula, a table, or the like. Further, the withdrawal timing determination unit 47 can quantitatively determine whether the withdrawal timing of the extracorporeal circulator 1 is appropriate. That is, the cardiac function measurement system 10 can quantitatively measure output ability of a patient. Accordingly, the cardiac function measurement system 10 according to the present specific example can easily determine the recovery of the cardiac function of the patient P with high accuracy.

The measured blood transfer flow rate determined by the measured blood transfer flow rate determination unit 45 is the average flow rate indicating the average value of the plurality of realistic flow rates measured by the flow rate measurement unit 21 in the predetermined time, and thus, the withdrawal timing determination unit 47 can determine the withdrawal timing of the extracorporeal circulator 1 by comparing the withdrawal reference flow rate and the measured blood transfer flow rate with each other and quantitatively measuring the output ability of the patient P while preventing an influence of pulsations of the heart P1 having a relatively large temporal change. Accordingly, the cardiac function measurement system 10 according to the present specific example can determine the recovery of the cardiac function of the patient P with higher accuracy.

In the first specific example described above with reference to Fig. 9, the display processing unit 41 displays, on the external monitor 16, the graph representing the relationship between the rotation speed of the centrifugal pump 3 and the withdrawal reference flow rate and the measured blood transfer flow rate. In the second specific example described above with reference to Fig. 10, the display processing unit 41 displays, on the external monitor 16, the graph representing the relationship between the parameter calculated by the parameter calculation unit 46 and the elapsing time, and the parameter calculated by the parameter calculation unit 46. According to the above, by checking the external monitor 16, the operator or the like can easily compare the withdrawal reference flow rate and the measured blood transfer flow rate, and can visually grasp the withdrawal timing of the extracorporeal circulator 1.

When the withdrawal timing of the extracorporeal circulator 1 is appropriate, the notification processing unit 42 notifies on the external monitor 16 that the withdrawal timing of the extracorporeal circulator 1 is appropriate. Accordingly, the operator or the like can visually and easily grasp whether the withdrawal timing of the extracorporeal circulator 1 is appropriate by checking the notification displayed on the external monitor 16. Accordingly, the cardiac function measurement system 10 according to the present specific example can prevent the withdrawal timing of the extracorporeal circulator 1 from being too earlier than the recovery of the cardiac function of the patient P, and can efficiently assist the determination of the withdrawal timing of the extracorporeal circulator 1.

When the predetermined time elapses after the notification processing unit 42 notifies on the external monitor 16 that the withdrawal timing of the extracorporeal circulator 1 is appropriate, the notification processing unit 42 notifies on the external monitor 16 that the timing at which the extracorporeal circulator 1 is withdrawn from the patient P is late. Accordingly, the operator or the like can visually and easily grasp whether the withdrawal timing of the extracorporeal circulator 1 is late by checking the notification displayed on the external monitor 16. Accordingly, the cardiac function measurement system 10 according to the present specific example can prevent the withdrawal timing of the extracorporeal circulator 1 from being too later than the recovery of the cardiac function of the patient P, and can efficiently assist the determination of the withdrawal timing of the extracorporeal circulator 1.

Next, experiments conducted by the present inventors will be described with reference to the drawings.

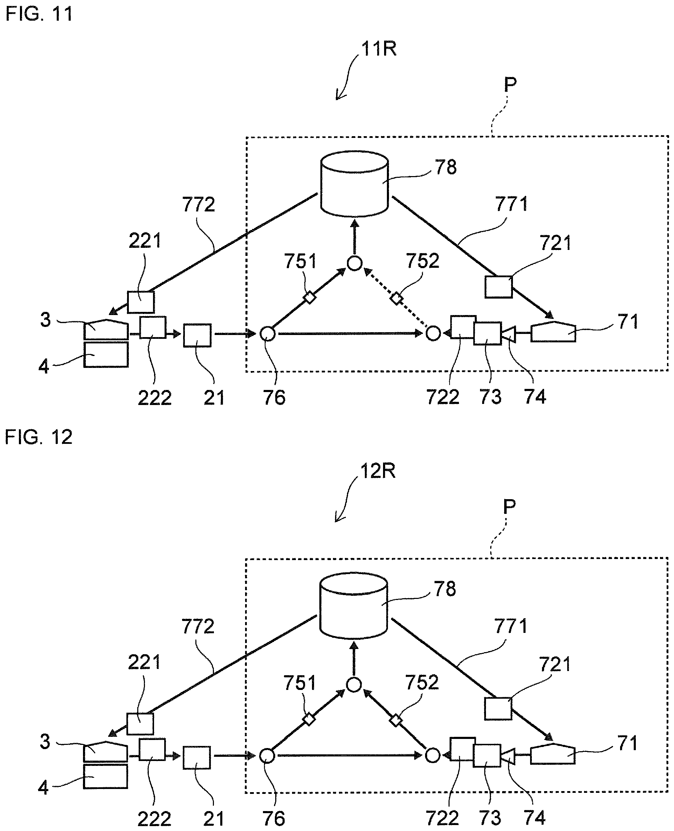

Fig. 11 is a block diagram illustrating a first circulation system set in a present experiment.

Fig. 12 is a block diagram illustrating a second circulation system set in the present experiment.

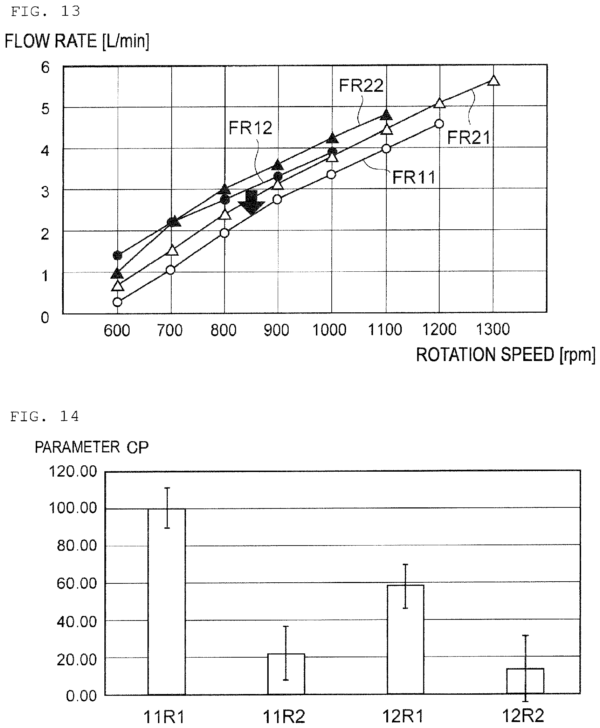

Fig. 13 is a graph showing an example of a relationship between a rotation speed of the centrifugal pump and a measured blood transfer flow rate that are obtained in the present experiment.

Fig. 14 is a graph showing an example of the parameter CP calculated by the parameter calculation unit in the present experiment.

The present inventors set a circulation system imitating a blood vessel of a human body and the circulation circuit 1R (see Fig. 1) using a tube made of a highly transparent, elastically deformable, and flexible synthetic resin such as vinyl chloride resin or silicone rubber. Arrows shown in a first circulation system 11R shown in Fig. 11 and a second circulation system 12R shown in Fig. 12 represent flows of a liquid imitating blood, and also represent the blood vessel of the human body and tubes imitating the blood removal side catheter 5, the blood removal tube 11, the blood transfer tube 12, and the blood transfer side catheter 6.

Lengths of the tubes are each set to approximately 15 cm or more and 130 cm or less for convenience of the experiment. The first circulation system 11R and the second circulation system 12R are disposed at positions having substantially the same height. Accordingly, a drop pressure does not necessarily need to be taken into consideration.

Each of the first circulation system 11R and the second circulation system 12R includes a pump 71, a first pressure sensor 721, a second pressure sensor 722, a flow rate sensor 73, a check valve 74, a first clamp 751, a second clamp 752, a bifurcated portion 76, a buffer tank 78, the centrifugal pump 3, the drive motor 4, a third pressure sensor 221, a fourth pressure sensor 222, and the flow rate measurement unit 21.

The pump 71 is a pump imitating the heart P1 of the patient P, is appropriately controlled to reproduce the pulsations of the heart P1 of the patient P, and is alternately driven at a relatively high rotation speed (that is, high discharge pressure) and a relatively low rotation speed (that is, low discharge pressure). In a pattern assuming that the heart of the patient P is healthy and strong, 1000 RPM is set as the high rotation speed of the pump 71, and 800 RPM is set as the low rotation speed of the pump 71. On the other hand, in a pattern assuming that the heart P1 of the patient P is not healthy and is weak, 800 RPM is set as the high rotation speed of the pump 71, and 400 RPM is set as the low rotation speed of the pump 71.

A drive time of the pump 71 at a high rotation speed is 0.5 seconds. A drive time of the pump 71 at a low rotation speed is 0.5 seconds. That is, in the present experiment, a cycle of the pump 71 is 1 second. In other words, the pump 71 is appropriately controlled to reproduce the pulsations of the heart P1 of the patient P, and is driven with a pulsation number of 60 bpm.

The first pressure sensor 721 is disposed in a tube 771 imitating a path through which the blood flows from a vena cava toward the heart P1 of the patient P, and detects a pressure of the liquid flowing through the tube 771. The second pressure sensor 722 detects a pressure of the liquid transferred from the pump 71. The flow rate sensor 73 detects a flow rate of the liquid transferred from the pump 71. The first clamp 751 is an adjustment tool that reproduces peripheral resistance on a lower limb side of the patient P. The second clamp 752 is an adjustment tool that reproduces peripheral resistance on a head side of the patient P. The bifurcated portion 76 is a portion imitating an insertion portion of the blood transfer side catheter 6 with respect to the femoral artery.

The third pressure sensor 221 is disposed in a tube 772 imitating the blood removal tube 11 as a path through which the blood removed from the femoral vein of the patient P flows toward the centrifugal pump 3, and detects a pressure of the liquid flowing through the tube 772. The fourth pressure sensor 222 detects a pressure of the liquid transferred from the centrifugal pump 3. The centrifugal pump 3, the drive motor 4, and the flow rate measurement unit 21 are as described above with reference to Fig. 1.

In the first circulation system 11R shown in Fig. 11, the present inventors adjusted an opening degree of the first clamp 751 such that a measurement result of the flow rate sensor 73 becomes approximately 4 L/min when the rotation speed of the pump 71 is 1000 RPM (that is, the discharge pressure of the pump 71 is approximately 100 mmHg) in a state in which the second clamp 752 is closed. On the other hand, in the second circulation system 12R shown in Fig. 12, the present inventors adjusted opening degrees of the first clamp 751 and the second clamp 752 such that a flow rate of the liquid flowing through each of a tube in which the first clamp 751 is disposed and a tube in which the second clamp 752 is disposed becomes approximately 2 L/min when the rotation speed of the pump 71 is 1000 RPM (that is, the discharge pressure of the pump 71 is approximately 100 mmHg).

The present inventors set the rotation speed of the centrifugal pump 3 in a range of approximately 600 RPM or more and 1200 RPM or less, and recorded a flow rate value of the liquid transferred from the centrifugal pump 3. The recorded flow rate value is an average value of a plurality of pieces of measurement data measured for one second by the flow rate measurement unit 21.

An example of results of the present experiment is as shown in Figs. 13 and 14. That is, Fig. 13 is a graph obtained as one example of the results of the present experiment, and is a graph illustrating an example of a relationship between the rotation speed of the centrifugal pump 3 and the flow rate value of the liquid transferred from the centrifugal pump 3 (that is, the average value of the plurality of pieces of measurement data measured for one second by the flow rate measurement unit 21). A horizontal axis of the graph shown in Fig. 13 represents the rotation speed (rpm) of the centrifugal pump 3. A vertical axis of the graph shown in Fig. 13 represents the flow rate value (L/min) of the liquid transferred from the centrifugal pump 3. That is, the graph shown in Fig. 13 corresponds to the graph described above with reference to Fig. 9.

A line FR11 shown in Fig. 13 is an example of an experimental result of a pattern assuming a case in which the heart of the patient P is healthy and strong in the first circulation system 11R. A line FR12 shown in Fig. 13 is an example of an experimental result of a pattern assuming a case in which the heart of the patient P is not healthy and is weak in the first circulation system 11R. A line FR21 shown in Fig. 13 is an example of an experimental result of a pattern assuming a case in which the heart of the patient P is healthy and strong in the second circulation system 12R. A line FR22 shown in Fig. 13 is an example of an experimental result of a pattern assuming a case in which the heart of the patient P is not healthy and is weak in the second circulation system 12R.

According to the graph shown in Fig. 13, in each of the first circulation system 11R and the second circulation system 12R, the lines FR11, FR21 of the experimental result of the pattern assuming the case in which the heart of the patient P is healthy and strong are located below the lines FR12, FR22 of the experimental result of the pattern assuming the case in which the heart of the patient P is not healthy and is weak. That is, as indicated by an arrow shown in Fig. 13, when the cardiac function of the patient P is recovered, the mark representing the relationship between the rotation speed of the centrifugal pump 3 and the flow rate value of the liquid transferred from the centrifugal pump 3 moves downward. Therefore, as described above with reference to Fig. 9, it was checked in the present experiment that, when the cardiac function of the patient P is recovered, the mark representing the relationship between the rotation speed of the centrifugal pump 3 and the measured blood transfer flow rate moves downward and approaches the line FR1 serving as the approximate line related to the withdrawal reference flow rate characteristic.

Fig. 14 is a graph showing an example of the parameter CP in each assumed pattern of the present experiment. A horizontal axis of the graph shown in Fig. 14 represents the assumed pattern of the present experiment. A vertical axis of the graph shown in Fig. 14 represents the parameter CP calculated by the parameter calculation unit 46. The parameter CP is as described above with reference to Fig. 10, and is the parameter calculated using, for example, Equation (1). The parameter CP shown in Fig. 14 is an average value of a plurality of values calculated using Equation (1) based on a plurality of rotation speeds of the centrifugal pump 3 and a plurality of flow rate values of the liquid transferred from the centrifugal pump 3.

That is, the parameter CP of the pattern 11R1 assuming the case in which the heart of the patient P is healthy and strong in the first circulation system 11R is an average value of a plurality of values calculated based on seven pieces of measurement data (rotation speed and flow rate) on the line FR11 shown in Fig. 13. The parameter CP of the pattern 11R2 assuming the case in which the heart of the patient P is not healthy and is weak in the first circulation system 11R is an average value of a plurality of values calculated based on five pieces of measurement data on the line FR12 shown in Fig. 13. The parameter CP of the pattern 12R1 assuming the case in which the heart of the patient P is healthy and strong in the second circulation system 12R is an average value of a plurality of values calculated based on eight pieces of measurement data on the line FR21 shown in Fig. 13. The parameter CP of the pattern 12R2 assuming the case in which the heart of the patient P is not healthy and is weak in the second circulation system 12R is an average value of a plurality of values calculated based on six pieces of measurement data on the line FR22 shown in Fig. 13.

According to the graph shown in Fig. 14, it was checked in the present experiment that, in both of the first circulation system 11R and the second circulation system 12R, the parameters CP of the patterns 11R1, 12R1 assuming the case in which the heart of the patient P is healthy and strong have a correlation higher than that of the parameters CP of the patterns 11R2, 12R2 assuming the case in which the heart of the patient P is not healthy and is weak.

The equation used for calculating the parameter CP is not limited to Equation (1) described above with reference to Fig. 10, and may be another equation. For example, the equation for calculating the parameter CP may be appropriately set such that the parameter CP in the case in which the heart of the patient P is healthy and strong has a correlation lower than that of the parameter CP in the case where the heart of the patient P is not healthy and is weak.

The embodiment according to this disclosure has been described above. However, the invention is not limited to the above-described embodiment, and various modifications can be made without departing from the scope of the invention which is solely defined by the appended claims.

Reference Signs List

1: extracorporeal circulator, 1R: circulation circuit, 2: oxygenator, 3: centrifugal pump, 4: drive motor, 5: blood removal side catheter, 6: blood transfer side catheter, 8, 9: connector, 10: cardiac function measurement system, 11: blood removal tube, 11R: first circulation system, 12: blood transfer tube, 12R: second circulation system, 14: oxygen supply tube, 16: external monitor, 21: flow rate measurement unit, 30: storage unit, 31: program, 32: withdrawal reference flow rate storage unit, 33: pump characteristic storage unit, 40: control unit, 41: display processing unit, 42: notification processing unit, 43: pump discharge pressure determination unit, 44: withdrawal reference flow rate determination unit, 45: measured blood transfer flow rate determination unit, 46: parameter calculation unit, 47: withdrawal timing determination unit, 48: CPU, 49: FPGA, 51: computer, 52: touch panel, 53: communication unit, 61: graph, 62: measured blood transfer flow rate, 63: rotation speed of centrifugal pump, 64: Index, 71: pump, 73: flow rate sensor, 74: check valve, 76: bifurcated portion, 78: buffer tank, 221: third pressure sensor, 222: fourth pressure sensor, 721: first pressure sensor, 722: second pressure sensor, 751: first clamp, 752: second clamp, 771, 772: tube, CP: parameter, G: signal, P: patient, P1: heart

**Claims**

1. A cardiac function measurement system (10) that is disposed in an extracorporeal circulator (1) and that is configured to measure a cardiac function of a patient (P), wherein

   the cardiac function measurement system (10) is configured

   to detect a rotation speed of a pump (71) configured to remove blood from the patient (P) and return the blood to the patient (P),
   to determine a discharge pressure of the pump (71) based on the rotation speed,
   to determine a withdrawal reference flow rate indicating a theoretical flow rate of the blood transferred from the pump (71) based on the discharge pressure,

   **characterized in that**
   said cardiac function measurement system is also configured

   to determine a measured blood transfer flow rate (62) indicating a realistic flow rate of the blood based on a measurement result of a flow rate measurement unit (21) configured to measure a flow rate of the blood flowing through a circulation circuit (1R) of the extracorporeal circulator (1),
   calculate a difference parameter (CP) representing a difference between the withdrawal reference flow rate and the measured blood transfer flow rate (62),

   determine the withdrawal timing of the extracorporeal circulator (1) based on the parameter (CP), and when the withdrawal timing is determined, a notification that the withdrawal timing is appropriate is displayed on a display unit.

2. The cardiac function measurement system (10) according to claim 1, wherein
   a graph (61) representing a relationship between the parameter (CP) and an elapsing time and the parameter (CP) are displayed on the display unit.

3. The cardiac function measurement system (10) according to any one of claims 1 or 2, wherein
   a graph (61) representing a relationship between the rotation speed, and the withdrawal reference flow rate and the measured blood transfer flow rate (62) is displayed on the display unit.

4. The cardiac function measurement system (10) according to claim 3, wherein
   when a predetermined time elapses after the notification that the withdrawal timing is appropriate is displayed on the display unit, a notification that the withdrawal timing is late is displayed on the display unit.

5. The cardiac function measurement system (10) according to any one of claims 1 to 4, wherein
   the measured blood transfer flow rate (62) is an average flow rate indicating an average value of a plurality of the realistic flow rates measured by the flow rate measurement unit (21) in a predetermined time.

6. An extracorporeal circulator (1) configured to perform extracorporeal circulation of blood using a circulation circuit (1R), the extracorporeal circulator (1) comprising:

   a blood removal side catheter (6) configured to be partially inserted into a patient (P) and guide the blood removed from the patient (P);
   a pump (71) configured to remove blood from the patient (P) and return the blood to the patient (P);
   a blood transfer side catheter that is disposed downstream of the pump (71), and that is configured to be partially inserted into the patient (P) and guide the blood transferred from the pump (71) to the patient (P);
   a flow rate measurement unit (21) that is disposed in the circulation circuit (1R) and that is configured to measure a flow rate of the blood flowing through the circulation circuit (1R); and
   the cardiac function measurement system (10) according to any one of claims 1 to 5.

7. A cardiac function measurement program executed by a computer of a cardiac function measurement system (10), the cardiac function measurement system (10) being disposed in an extracorporeal circulator (1) and being configured to measure a cardiac function of a patient (P), the cardiac function measurement program causing the computer to execute steps of:

detecting a rotation speed of a pump (71) configured to remove blood from the patient (P) and return the blood to the patient (P);

determining a discharge pressure of the pump (71) based on the rotation speed;

determining a withdrawal reference flow rate indicating a theoretical flow rate of the blood transferred from the pump (71) based on the discharge pressure;

determining a measured blood transfer flow rate (62) indicating a realistic flow rate of the blood based on a measurement result of a flow rate measurement unit (21) configured to measure a flow rate of the blood flowing through a circulation circuit (1R) of the extracorporeal circulator (1); and

determining a withdrawal timing of the extracorporeal circulator (1) based on a comparison between the withdrawal reference flow rate and the measured blood transfer flow rate (62);

calculating a difference parameter (CP) representing a difference between the withdrawal reference flow rate and the measured blood transfer flow rate (62); determining the withdrawal timing of the extracorporeal circulator (1) based on the parameter (CP), and displaying a notification that the withdrawal timing is appropriate on a display unit when the withdrawal timing is appropriate.

**Patentansprüche**

1. Herzfunktionsmesssystem (10), das in einem extrakorporalen Zirkulator (1) angeordnet und so konfiguriert ist, dass es eine Herzfunktion eines Patienten (P) misst, wobei

das Herzfunktionsmesssystem (10) konfiguriert ist, um

eine Rotationsgeschwindigkeit einer Pumpe (71) zu erfassen, die so konfiguriert ist, dass sie Blut von dem Patienten (P) entnimmt und das Blut zu dem Patienten (P) zurückführt,

einen Ausgabedruck der Pumpe (71) auf der Grundlage der Rotationsgeschwindigkeit zu bestimmen,

eine Referenz-Durchflussmenge zur Entfernung zu bestimmen, die eine theoretische Durchflussmenge des von der Pumpe (71) übertragenen Blutes auf der Grundlage des Ausgabedrucks angibt,

**dadurch gekennzeichnet, dass**

das Herzfunktionsmesssystem ebenfalls konfiguriert ist, um

eine gemessene Blutübertragungs-Durchflussmenge (62) zu bestimmen, die eine realistische Durchflussmenge des Blutes auf der Grundlage eines Messergebnisses einer Durchflussmengen-Messeinheit (21) angibt, die konfiguriert ist, um eine Durchflussmenge des Blutes zu messen, das durch einen Zirkulationskreislauf (1R) des extrakorporalen Zirkulators (1) strömt,

einen Differenzparameter (CP) zu bestimmen, der eine Differenz zwischen der Referenz-Durchflussmenge zur Entfernung und der gemessenen Blutübertragungs-Durchflussmenge (62) darstellt,

eine zeitliche Regulierung zur Entfernung des extrakorporalen Zirkulators (1) auf der Grundlage des Parameters (CP) zu bestimmen, und wenn die zeitliche Regulierung zur Entfernung bestimmt ist, wird eine Mitteilung, dass die zeitliche Regulierung zur Entfernung angemessen ist, auf einer Anzeigeeinheit angezeigt.

2. Herzfunktionsmesssystem (10) nach Anspruch 1, wobei eine grafische Darstellung (61), die eine Beziehung zwischen dem Parameter (CP) und einer verstreichenden Zeit darstellt, und der Parameter (CP) auf der Anzeigeeinheit angezeigt werden.

3. Herzfunktionsmesssystem (10) nach einem der Ansprüche 1 oder 2, wobei eine grafische Darstellung (61), die eine Beziehung zwischen der Rotationsgeschwindigkeit, der Referenz-Durchflussmenge zur Entfernung und der gemessenen Blutübertragungs-Durchflussmenge (62) darstellt, auf der Anzeigeeinheit angezeigt wird.

4. Herzfunktionsmesssystem (10) nach Anspruch 3, wobei wenn eine vorbestimmte Zeit verstreicht, nachdem die Meldung, dass die zeitliche Regulierung zur Entfernung angemessen ist, auf der Anzeigeeinheit angezeigt wird, eine Meldung, dass die zeitliche Regulierung zur Entfernung verspätet ist, auf der Anzeigeeinheit angezeigt wird.

5. Herzfunktionsmesssystem (10) nach einem der Ansprüche 1 bis 4, wobei die gemessene Blutübertragungs-Durchflussmenge (62) eine durchschnittliche Durchflussmenge ist, die einen Durchschnittswert einer Vielzahl von realistischen Durchflussmengen angibt, die von der Durchflussmengen-Messeinheit (21) in einer vorbestimmten Zeit gemessen wurden.

6.  Extrakorporaler Zirkulator (1), der konfiguriert ist, um eine extrakorporale Zirkulation von Blut unter Verwendung eines Zirkulationskreislaufs (1R) durchzuführen, wobei der extrakorporale Zirkulator (1) umfasst:

    einen Katheter (6) der Blutentnahmeseite, der so konfiguriert ist, dass er teilweise in einen Patienten (P) eingeführt wird und das aus dem Patienten (P) entnommene Blut leitet;
    eine Pumpe (71), die so konfiguriert ist, dass sie dem Patienten (P) Blut entnimmt und das Blut zu dem Patienten (P) zurückführt;
    einen Katheter der Blutübertragungsseite, der stromabwärts von der Pumpe (71) angeordnet ist und der so konfiguriert ist, dass er teilweise in den Patienten (P) eingeführt wird und das von der Pumpe (71) zu dem Patienten (P) übertragene Blut leitet;
    eine Durchflussmengen-Messeinheit (21), die in dem Zirkulationskreislauf (1R) angeordnet ist und die so konfiguriert ist, dass sie eine Durchflussmenge des durch den Zirkulationskreislauf (1R) strömenden Blutes misst; und
    das Herzfunktionsmesssystem (10) nach einem der Ansprüche 1 bis 5.

7.  Herzfunktionsmessprogramm, das von einem Computer eines Herzfunktionsmesssystems (10) ausgeführt wird, wobei das Herzfunktionsmesssystem (10) in einem extrakorporalen Zirkulator (1) angeordnet und so konfiguriert ist, dass es eine Herzfunktion eines Patienten (P) misst, wobei das Herzfunktionsmessprogramm den Computer veranlasst, folgende Schritte auszuführen:

    Erfassen einer Rotationsgeschwindigkeit einer Pumpe (71), die so konfiguriert ist, dass sie dem Patienten (P) Blut entnimmt und das Blut zu dem Patienten (P) zurückführt;
    Bestimmen eines Ausgabedrucks der Pumpe (71) auf der Grundlage der Rotationsgeschwindigkeit;
    Bestimmen einer Referenz-Durchflussmenge zur Entfernung, die eine theoretische Durchflussmenge des von der Pumpe (71) übertragenen Blutes auf der Grundlage des Ausgabedrucks angibt;
    Bestimmen einer gemessenen Blutübertragungs-Durchflussmenge (62), die eine realistische Durchflussmenge des Blutes auf der Grundlage eines Messergebnisses einer Durchflussmengen-Messeinheit (21) angibt, die konfiguriert ist, um eine Durchflussmenge des Blutes zu messen, das durch einen Zirkulationskreislauf (1R) des extrakorporalen Zirkulators (1) strömt; und
    Bestimmen einer zeitlichen Regulierung zur Entfernung des extrakorporalen Zirkulators (1) auf der Grundlage eines Vergleichs zwischen der Referenz-Durchflussmenge zur Entfernung und der gemessenen Blutübertragungs-Durchflussmenge (62);
    Berechnen eines Differenzparameters (CP), der eine Differenz zwischen der Referenz-Durchflussmenge zur Entfernung und der gemessenen Blutübertragungs-Durchflussmenge (62) darstellt;
    Bestimmen der zeitlichen Regulierung zur Entfernung des extrakorporalen Zirkulators (1) auf der Grundlage des Parameters (CP), und Anzeigen einer Mitteilung, dass die zeitliche Regulierung zur Entfernung angemessen ist, auf einer Anzeigeeinheit, wenn die zeitliche Regulierung zur Entfernung angemessen ist.

**Revendications**

1.  Système de mesure de fonction cardiaque (10) qui est disposé dans un circulateur extracorporel (1) et qui est configuré pour mesurer une fonction cardiaque d'un patient (P), dans lequel le système de mesure de fonction cardiaque (10) est configuré pour

    détecter une vitesse de rotation d'une pompe (71) configurée pour prélever du sang du patient (P) et retourner le sang au patient (P),
    déterminer une pression de décharge de la pompe (71) sur la base de la vitesse de rotation,
    déterminer un débit de référence de retrait indiquant un débit théorique du sang transféré de la pompe (71) sur la base de la pression de décharge,
    **caractérisé en ce que**
    ledit système de mesure de fonction cardiaque est également configuré pour
    déterminer un débit de transfert de sang mesuré (62) indiquant un débit réaliste du sang sur la base d'un résultat de mesure d'une unité de mesure de débit (21) configurée pour mesurer un débit du sang s'écoulant à travers un circuit de circulation (1R) du circulateur extracorporel (1),
    calculer un paramètre de différence (CP) représentant une différence entre le débit de référence de retrait et le débit de transfert de sang mesuré (62),
    déterminer la temporisation de retrait du circulateur extracorporel (1) sur la base du paramètre (CP) et, lorsque la temporisation de retrait est déterminée, une notification que la temporisation de retrait est appropriée est affichée sur une unité d'affichage.

**2.** Système de mesure de fonction cardiaque (10) selon la revendication 1, dans lequel
un graphe (61) représentant une relation entre le paramètre (CP) et un temps qui s'écoule et le paramètre (CP) sont affichés sur l'unité d'affichage.

**3.** Système de mesure de fonction cardiaque (10) selon l'une quelconque des revendications 1 ou 2, dans lequel un graphe (61) représentant une relation entre la vitesse de rotation, et le débit de référence de retrait et le débit de transfert de sang mesuré (62) est affiché sur l'unité d'affichage.

**4.** Système de mesure de fonction cardiaque (10) selon la revendication 3, dans lequel
lorsqu'un temps prédéterminé s'écoule après l'affichage, sur l'unité d'affichage, de la notification que la temporisation de retrait est appropriée, une notification que la temporisation de retrait est en retard est affichée sur l'unité d'affichage.

**5.** Système de mesure de fonction cardiaque (10) selon l'une quelconque des revendications 1 à 4, dans lequel le débit de transfert de sang mesuré (62) est un débit moyen indiquant une valeur moyenne d'une pluralité des débits réalistes mesurés par l'unité de mesure de débit (21) au cours d'un temps prédéterminé.

**6.** Circulateur extracorporel (1) configuré pour réaliser une circulation extracorporelle de sang à l'aide d'un circuit de circulation (1R), le circulateur extracorporel (1) comprenant :

un cathéter côté prélèvement de sang (6) configuré pour être partiellement inséré dans un patient (P) et guider le sang prélevé du patient (P) ;
une pompe (71) configurée pour prélever du sang du patient (P) et retourner le sang au patient (P) ;
un cathéter côté transfert de sang qui est disposé en aval de la pompe (71), et qui est configuré pour être partiellement inséré dans le patient (P) et guider le sang transféré de la pompe (71) vers le patient (P) ;
une unité de mesure de débit (21) qui est disposée dans le circuit de circulation (1R) et qui est configurée pour mesurer un débit du sang s'écoulant à travers le circuit de circulation (1R) ; et
le système de mesure de fonction cardiaque (10) selon l'une quelconque des revendications 1 à 5.

**7.** Programme de mesure de fonction cardiaque exécuté par un ordinateur d'un système de mesure de fonction cardiaque (10), le système de mesure de fonction cardiaque (10) étant disposé dans un circulateur extracorporel (1) et étant configuré pour mesurer une fonction cardiaque d'un patient (P), le programme de mesure de fonction cardiaque amenant l'ordinateur à exécuter les étapes consistant à :

détecter une vitesse de rotation d'une pompe (71) configurée pour prélever du sang du patient (P) et retourner le sang au patient (P) ;
déterminer une pression de décharge de la pompe (71) sur la base de la vitesse de rotation ;
déterminer un débit de référence de retrait indiquant un débit théorique du sang transféré de la pompe (71) sur la base de la pression de décharge ;
déterminer un débit de transfert de sang mesuré (62) indiquant un débit réaliste du sang sur la base d'un résultat de mesure d'une unité de mesure de débit (21) configurée pour mesurer un débit du sang s'écoulant à travers un circuit de circulation (1R) du circulateur extracorporel (1) ; et
déterminer une temporisation de retrait du circulateur extracorporel (1) sur la base d'une comparaison entre le débit de référence de retrait et le débit de transfert de sang mesuré (62) ;
calculer un paramètre de différence (CP) représentant une différence entre le débit de référence de retrait et le débit de transfert de sang mesuré (62) ;
déterminer la temporisation de retrait du circulateur extracorporel (1) sur la base du paramètre (CP) ; et
afficher, sur une unité d'affichage, une notification que la temporisation de retrait est appropriée, lorsque la temporisation de retrait est appropriée.

FIG. 1

FIG. 2

FIG. 3

FIG. 4

FIG. 5

CARDIAC FUNCTION
MEASUREMENT SYSTEM

1

10

40

30

41 — DISPLAY PROCESSING UNIT

42 — NOTIFICATION PROCESSING UNIT

43 — PUMP DISCHARGE PRESSURE
DETERMINATION UNIT

44 — WITHDRAWAL REFERENCE FLOW
RATE DETERMINATION UNIT

45 — MEASURED BLOOD TRANSFER
FLOW RATE DETERMINATION UNIT

46 — PARAMETER CALCULATION UNIT

47 — WITHDRAWAL TIMING
DETERMINATION UNIT

CONTROL UNIT

WITHDRAWAL
REFERENCE FLOW
RATE STORAGE UNIT — 32

PUMP
CHARACTERISTIC
STORAGE UNIT — 33

STORAGE UNIT

52 — TOUCH PANEL
(DISPLAY UNIT)

53 — COMMUNICATION UNIT

16 — EXTERNAL MONITOR
(DISPLAY UNIT)

DRIVE MOTOR — 4

FLOW RATE
MEASUREMENT UNIT — 21

FIG. 6

FIG. 7

```
                        ┌─────────────┐
                        │    START    │
                        └─────────────┘
                               │
S11 ┌──────────────────────────────────────────────────────────────┐
    │         RECEIVE SIGNAL RELATED TO ROTATION SPEED OF           │
    │         CENTRIFUGAL PUMP FROM CENTRIFUGAL PUMP                │
    └──────────────────────────────────────────────────────────────┘
                               │
S12 ┌──────────────────────────────────────────────────────────────┐
    │  RECEIVE SIGNAL RELATED TO FLOW RATE OF BLOOD TRANSFERRED FROM │
    │  CENTRIFUGAL PUMP FROM FLOW RATE MEASUREMENT UNIT             │
    └──────────────────────────────────────────────────────────────┘
                               │
S13 ┌──────────────────────────────────────────────────────────────┐
    │       DETERMINE DISCHARGE PRESSURE OF CENTRIFUGAL PUMP         │
    │       BASED ON ROTATION SPEED OF CENTRIFUGAL PUMP             │
    └──────────────────────────────────────────────────────────────┘
                               │
S14 ┌──────────────────────────────────────────────────────────────┐
    │      DETERMINE WITHDRAWAL REFERENCE FLOW RATE INDICATING       │
    │ THEORETICAL FLOW RATE OF BLOOD TRANSFERRED FROM CENTRIFUGAL PUMP│
    │       BASED ON DISCHARGE PRESSURE OF CENTRIFUGAL PUMP         │
    └──────────────────────────────────────────────────────────────┘
                               │
S15 ┌──────────────────────────────────────────────────────────────┐
    │        DETERMINE, BASED ON MEASUREMENT RESULT OF FLOW RATE     │
    │ MEASUREMENT UNIT, MEASURED BLOOD TRANSFER FLOW RATE INDICATING │
    │ REALISTIC FLOW RATE OF BLOOD TRANSFERRED FROM CENTRIFUGAL PUMP │
    └──────────────────────────────────────────────────────────────┘
                               │
S16 ┌──────────────────────────────────────────────────────────────┐
    │  CALCULATE PARAMETER INDICATING COMPARISON BETWEEN WITHDRAWAL  │
    │  REFERENCE FLOW RATE AND MEASURED BLOOD TRANSFER FLOW RATE    │
    └──────────────────────────────────────────────────────────────┘
                               │
S17 ┌──────────────────────────────────────────────────────────────┐
    │   DISPLAY, ON DISPLAY UNIT, INFORMATION ON PARAMETER INDICATING│
    │   COMPARISON BETWEEN WITHDRAWAL REFERENCE FLOW RATE AND       │
    │         MEASURED BLOOD TRANSFER FLOW RATE                     │
    └──────────────────────────────────────────────────────────────┘
                               │
S18 ╱────────────────────────────────────────────────────────────────╲
   ╱ DETERMINE WHETHER WITHDRAWAL TIMING OF EXTRACORPOREAL CIRCULATOR IS╲ NO
   ╲ APPROPRIATE BASED ON PARAMETER INDICATING COMPARISON BETWEEN WITHDRAWAL╱──
    ╲   REFERENCE FLOW RATE AND MEASURED BLOOD TRANSFER FLOW RATE      ╱
                               │ YES
                         ┌──────────┐
                         │    1     │
                         └──────────┘
```

25

FIG. 8

```
                              ┌─┐
                              │1│
                              └┬┘                              ┌───┐
                               │                               │ 2 │
                               ▼                               └─┬─┘
   S19 ┌─────────────────────────────────────────────────────┐  │
       │ NOTIFY THAT WITHDRAWAL TIMING OF EXTRACORPOREAL       │  │
       │ CIRCULATOR IS APPROPRIATE ON DISPLAY UNIT             │  │
       └─────────────────────────┬───────────────────────────┘  │
                                 │◄──────────────────────────────┘
   S20                           ▼                          NO
       ◄─── DETERMINE WHETHER EXTRACORPOREAL CIRCULATOR IS WITHDRAWN ───►
                                 │ YES                         │
                                 ▼                             ▼
                            ╭─────────╮                      ┌───┐
                            │   END   │                      │ 1 │
                            ╰─────────╯                      └───┘
```

```
                              ┌───┐
                              │ 1 │
                              └─┬─┘
                                ▼
   S21                                                       NO
       ◄─── DETERMINE WHETHER PREDETERMINED TIME ELAPSES AFTER NOTIFYING THAT ───►
            WITHDRAWAL TIMING OF EXTRACORPOREAL CIRCULATOR
            IS APPROPRIATE ON DISPLAY UNIT                         │
                                │ YES                              │
                                ▼                                  │
   S22 ┌──────────────────────────────────────────────────────┐   │
       │ NOTIFY THAT WITHDRAWAL TIMING OF EXTRACORPOREAL        │   │
       │ CIRCULATOR IS LATE ON DISPLAY UNIT                     │   │
       └──────────────────────────┬───────────────────────────┘   │
                                  │◄──────────────────────────────┘
                                  ▼
                               ┌───┐
                               │ 2 │
                               └───┘
```

FIG. 9

FIG. 10

FIG. 11

FIG. 12

FIG. 13

FIG. 14

29

**EP 3 932 302 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018353667 A1
- JP 2002224066 A